(19) Europäisches Patentamt
European Patent Office
Office européen des brevets

(11) EP 1 117 642 B1

(12) **EUROPÄISCHE PATENTSCHRIFT**

(45) Veröffentlichungstag und Bekanntmachung des Hinweises auf die Patenterteilung:
**26.05.2004 Patentblatt 2004/22**

(21) Anmeldenummer: **99948791.1**

(22) Anmeldetag: **18.09.1999**

(51) Int Cl.[7]: **C07D 213/74**, C07D 401/14, C07D 409/14, C07D 405/14, C07D 417/14, C07D 471/04, C07D 473/04, C07D 413/14, A61K 31/4427

(86) Internationale Anmeldenummer:
**PCT/EP1999/006933**

(87) Internationale Veröffentlichungsnummer:
**WO 2000/020393 (13.04.2000 Gazette 2000/15)**

(54) **SUBSTITUIERTE 1,3-DIARYL-2-PYRIDIN-2-YL-3-(PYRIDIN-2-YLAMINO)-PROPANOLDERIVATE, VERFAHREN ZU DEREN HERSTELLUNG, DIESE VERBINDUNGEN ENTHALTENDE ARZNEIMITTEL UND DEREN VERWENDUNG**

SUBSTITUTED 1,3-DIARYL-2-PYRIDINE-2-YL-3-(PYRIDINE-2-YLAMINO)-PROPANOL DERIVATIVES, METHODS FOR THEIR PRODUCTION, PHARMACEUTICAL COMPOSITIONS CONTAINING THE SAME AND THEIR USE

DERIVES DE 1,3-DIARYL-2-PYRIDINE-2-YL-3-(PYRIDINE-2-YLAMINO)PROPANOL SUBSTITUES, PROCEDES PERMETTANT DE LES PREPARER, MEDICAMENTS CONTENANT LESDITS COMPOSES ET LEUR UTILISATION

(84) Benannte Vertragsstaaten:
**AT BE CH CY DE DK ES FI FR GB GR IE IT LI LU NL PT SE**

(30) Priorität: **02.10.1998 DE 19845406**

(43) Veröffentlichungstag der Anmeldung:
**25.07.2001 Patentblatt 2001/30**

(73) Patentinhaber: **Aventis Pharma Deutschland GmbH**
**65929 Frankfurt am Main (DE)**

(72) Erfinder:
- **KIRSCH, Reinhard**
  **D-38100 Braunschweig (DE)**
- **ENHSEN, Alfons**
  **D-64572 Büttelborn (DE)**
- **GLOMBIK, Heiner**
  **D-65719 Hofheim (DE)**
- **KRAMER, Werner**
  **D-55130 Mainz-Laubenheim (DE)**
- **FALK, Eugen**
  **D-60529 Frankfurt am Main (DE)**

(56) Entgegenhaltungen:
**EP-A- 0 869 121**

- **Y H ET AL: "Hypolipidemic effects of alpha, beta, and gamma-alkylaminophenone analogs in rodents" EUROPEAN JOURNAL OF MEDICINAL CHEMISTRY.CHIMICA THERAPEUTICA,FR,EDITIONS SCIENTIFIQUE ELSEVIER, PARIS, Bd. 31, Nr. 4, Seite 281-290 XP004040088 ISSN: 0223-5234**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 117 642 B1

Printed by Jouve, 75001 PARIS (FR)

## Beschreibung

Die Erfindung betrifft substituierte 1,3-Diaryl-2-pyridin-2-yl-3-(pyridin-2-ylamino)-propanolderivate und deren pharmazeutisch verträgliche Salze und physiologisch funktionelle Derivate.

Es sind bereits mehrere Wirkstoffklassen zur Behandlung von Adipositas und von Lipidstoffwechselstörungen beschrieben worden:

- polymere Adsorber, wie z.B. Cholestyramin
- Benzothiazepine (WO 93/16055)
- Gallensäuredimere und -konjugate (EP 0 489 423)
- 4-Amino-2-ureido-pyrimidin-5-carbonsäureamide (EP 0 557 879)

Der Erfindung lag die Aufgabe zugrunde, weitere Verbindungen zur Verfügung zu stellen, die eine therapeutisch verwertbare hypolipidämische Wirkung entfalten.

Die Erfindung betrifft daher 1,3-Diaryl-2-pyridin-2-yl-3-(pyridin-2-ylamino)-propanolderivate der Formel I,

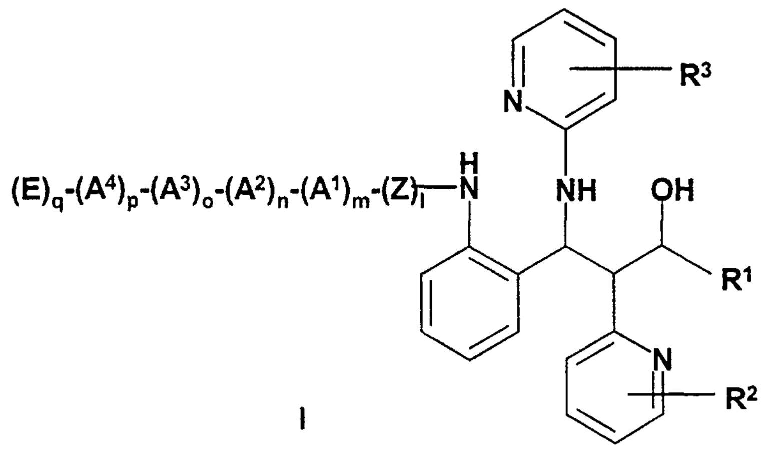

I

worin bedeuten

Z: -NH-($C_1$-$C_{16}$-Alkyl)-(C=O)-;
-(C=O)-($C_1$-$C_{16}$-Alkyl)-(C=O)- ;
- (C=O)-Phenyl-(C=O)- ;

$A^1$, $A^2$, $A^3$,$A^4$: unabhängig voneinander einen Aminosäurerest, einen Aminosäurerest der ein- oder mehrfach substituiert ist durch Aminosäure-Schutzgruppen;

E: -$SO_2$-$R^4$, -CO-$R^4$;

$R^1$: Phenyl, Thiazolyl, Oxazolyl, Thienyl, Thiophenyl, Furanyl, Pyridyl, Pirimidyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]$_2$, CONH($C_3$-$C_6$)Cycloalkyl, $NH_2$, NH-CO-($C_1$-$C_6$)-Alkyl, NH-CO-Phenyl;

$R^2$: H, OH, $CH_2OH$, OMe,

$R^3$: H, F, Methyl, OMe;

$R^4$: -($C_5$-$C_{16}$-Alkyl), -($C_0$-$C_{16}$-Alkylen)-$R^5$, -(C=O)-($C_0$-$C_{16}$-Alkylen)-$R^5$, -(C=O)-($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-S(O)$_{0-2}$-$R^5$, -($C_1$-$C_4$-Alkylen)-O-$R^5$, -($C_1$-$C_4$-Alkylen)-NH-$R^5$;

$R^5$ -COO-$R^6$, -(C=O)- $R^6$, -($C_1$-$C_6$-Alkylen)-$R^7$, -($C_1$-$C_6$-Alkenylen)-$R^7$ , (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]2, CONH($C_3$-$C_6$)Cycloalkyl, $NH_2$, NH-CO-($C_1$-$C_6$)-Alky, NH-CO-Phenyl, Pyridyl;

$R^6$ H, -($C_1$-$C_6$)Alkyl;

$R^7$ H, (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]2, CONH($C_3$-$C_6$)Cycloalkyl, $NH_2$, NH-CO-($C_1$-$C_6$)-Alky, NH-CO-Phenyl;

l, q, m, n, o, p unabhängig voneinander 0 oder 1, mit
l+q+m+n+o+p größer oder gleich 1;

sowie deren pharmazeutisch verträgliche Salze.

Bevorzugt sind Verbindungen der Formel I, in denen ein oder mehrere Rest(e) die folgende Bedeutung hat bzw. haben:

Z -NH-($C_1$-$C_{16}$-Alkyl)-(C=O)-,
-(C=O)-($C_1$-$C_{16}$-Alkyl)-(C=O)- ,
-(C=O)-Phenyl-(C=O)- ;

$A^1$, $A^2$, $A^3$,$A^4$, unabhängig voneinander einen Aminosäurerest, einen Aminosäurerest der ein- oder mehrfach substituiert ist durch Aminosäure-Schutzgruppen;

E -$SO_2$-$R^4$, -CO-$R^4$;

$R^1$ Phenyl, Thiazolyl, Oxazolyl, Thienyl, Thiophenyl, Furanyl, Pyridyl, Pirimidyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]2, CONH ($C_3$-$C_6$)Cycloalkyl, $NH_2$, NH-CO-($C_1$-$C_6$)-Alkyl, NH-CO-Phenyl,;

$R^2$ H, OH, $CH_2OH$, OMe;

$R^3$ H, F, Methyl, OMe;

$R^4$ -($C_5$-$C_{16}$-Alkyl), -($C_0$-$C_{16}$-Alkylen)-$R^5$, -(C=O)-($C_0$-$C_{16}$-Alkylen)-$R^5$, -(C=O)-($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-$S(O)_{0-2}$-$R^5$, -($C_1$-$C_4$-Alkylen)-O-$R^5$, -($C_1$-$C_4$-Alkylen)-NH-$R^5$;

$R^5$ -COO-$R^6$, -(C=O)- $R^6$ , -($C_1$-$C_6$-Alkylen)-$R^7$, -($C_1$-$C_6$-Alkenylen)-$R^7$ , -($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]2, CONH($C_3$-$C_6$)Cycloalkyl, $NH_2$, NH-CO-($C_1$-$C_6$)-Alkyl, NH-CO-Phenyl, Pyridyl;

$R^6$ H, -($C_1$-$C_6$)Alkyl;

$R^7$ H, (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]2, CONH($C_3$-$C_6$)Cycloalkyl, $NH_2$, NH-CO-($C_1$-$C_6$)-Alkyl, NH-CO-Phenyl;

l 0 oder 1;

m, n 0;

o 1;

p 0 oder 1;

q 0 oder 1;

sowie deren pharmazeutisch verträgliche Salze.

Besonders bevorzugt sind Verbindungen der Formel I, in denen ein oder mehrere Rest(e) die folgende Bedeutung hat bzw. haben:

Z -NH-($C_1$-$C_{12}$-Alkyl)-(C=O)-,
-(C=O)-($C_1$-$C_{12}$-Alkyl)-(C=O)- ,
-(C=O)-Phenyl-(C=O)- ;

$A^1$, $A^2$, $A^3$,$A^4$, unabhängig voneinander einen Aminosäurerest, einen Aminosäurerest der ein- oder mehrfach substituiert ist durch Aminosäure-Schutzgruppen;

E -$SO_2$-$R^4$, -CO-$R^4$;

$R^1$ Phenyl, Thiazolyl, Oxazolyl, wobei die Ringe bis zu 3 mal substituiert sein können mit -($C_1$-$C_6$)-Alkyl;

$R^2$ H, OH, $CH_2OH$, OMe;

$R^3$ H, F, Methyl, OMe;

$R^4$ -($C_5$-$C_{16}$-Alkyl), -($C_0$-$C_{16}$-Alkylen)-$R^5$, -(C=O)-($C_0$-$C_{16}$-Alkylen)-$R^5$, -(C=O)($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-S(0)$_{0-2}$-$R^5$, -($C_1$-$C_4$-Alkylen)-O-$R^5$, -($C_1$-$C_4$-Alkylen)-NH-$R^5$;

$R^5$ -COO-$R^6$, -(C=O)-$R^6$, -($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 2 mal substituiert sein können mit F, Cl, Br, OH, $CF_3$, $NO_2$, CN, $OCF_3$, -($C_1$-$C_6$)-Alkyl, O-($C_1$-$C_6$) -Alkyl, COOH, COO($C_1$-$C_6$)Alkyl, $CONH_2$, CONH($C_1$-$C_6$)Alkyl, CON[($C_1$-$C_6$)Alkyl]$_2$, CONH($C_3$-$C_6$) Cycloalkyl, $NH_2$, NH-CO-($C_1$-$C_6$)-Alkyl, NH-CO-Phenyl, Pyridyl;

$R^6$ H, -($C_1$-$C_6$)Alkyl;

l, m, n 0;

o 1;

p 0 oder 1;

q 0 oder 1;

sowie deren pharmazeutisch verträgliche Salze.

Unter dem Begriff Alkyl werden geradkettige oder verzweigte Kohlenwasserstoffketten verstanden.

Mit dem Begriff Aminosäuren bzw. Aminosäurereste sind z.B. die stereoisomeren Formen, d.h. D- oder L-Formen, folgender Verbindungen gemeint:

| | | |
|---|---|---|
| Alanin | Glycin | Prolin |
| Cystein | Histidin | Glutamin |
| Asparaginsäure | Isoleucin | Arginin |
| Glutaminsäure | Lysin | Serin |
| Phenylalanin | Leucin | Threonin |
| Tryptophan | Methionin | Valin |
| Tyrosin | Asparagin | |

| | |
|---|---|
| 2-Aminoadipinsäure | 2-Aminoisobuttersäure |
| 3-Aminoadipinsäure | 3-Aminoisobuttersäure |
| beta-Alanin | 2-Aminopimelinsäure |
| 2-Aminobuttersäure | 2,4-Diaminobuttersäure |
| 4-Aminobuttersäure | Desmosin |
| Piperidinsäure | 2,2-Diaminopimelinsäure |
| 6-Aminocapronsäure | 2,3-Diaminopropionsäure |
| 2-Aminoheptansäure | N-Ethylglycin |
| 2-(2-Thienyl)-glycin | 3-(2-Thienyl)-alanin |
| Penicillamin | Sarkosin |
| N-Ethylasparagin | N-Methylisoleucin |
| Hydroxylysin | 6-N-Methyllysin |
| allo-Hydroxylysin | N-Methylvalin |
| 3-Hydroxyprolin | Norvalin |
| 4-Hydroxyprolin | Norleucin |
| Isodesmosin | Ornithin |
| allo-Isoleucin | 3-(2-Naphthyl)-alanin |
| Azaglycin | N-Cyclohexylglycin |
| 2,4-Diaminobuttersäure | |

Die Kurzschreibweise der Aminosäuren erfolgte nach der allgemein üblichen Schreibweise (vgl. Schröder, Lübke, The Peptides, Band I, New York 1965, Seiten XXII-XXIII; Houben-Weyl, Methoden der Organischen Chemie, Band XV/1 und 2, Stuttgart 1974). Die Aminosäure pGlu steht für Pyroglutamyl, Nal für 3-(2-Naphthyl)-alanin, Azagly-$NH_2$ für eine Verbindung der Formel $NH_2$-NH-$CONH_2$ und D-Asp für die D-Form von Asparaginsäure. Peptide sind ihrer chemischen Natur nach Säureamide und zerfallen bei der Hydrolyse in Aminosäuren.

Die Erfindung betrifft weiterhin Verfahren zur Herstellung von Verbindungen der Formel I , die durch folgende Reaktionsschemata (Schema 1 bis 6) gekennzeichnet sind.

Die erfindungsgemäßen Verbindungen der Formel I werden ausgehend von Verbindungen der Formeln VI oder VII nach den allgemeinen Methoden der Peptidchemie stufenweise von der freien Aminogruppe her oder durch Kupplung von Segmenten hergestellt (Houben-Weyl, Methoden der Organischen Chemie, Band 15/1,2). Die Peptidkupplungen können z.B. mit TOTU (Literatur siehe: G. Breipohl, W. König EP 0460446; W. König, G. Breipohl, P. Pokorny, M. Birkner in E. Giralt and D. Andreu (Eds.) Peptides 1990, Escom, Leiden, 1991, 143-145) nach der Methode der gemischten Anhydride, über Aktivester, Azide oder nach der Carbodiimid-Methode, insbesondere unter Zusatz reaktionsbeschleunigender und racemisierungsverhindernder Substanzen, wie z.B. 1-Hydroxybenzotriazol, N-Hydroxysuccinimid, 3-Hydroxy-4-oxo-3.4-dihydro-1.2.3-benzotriazin, N-Hydroxy-5-norbornen-2.3-dicarboximid, ferner unter Verwendung aktiver Derivate des 1-Hydroxybenzotriazols oder Anhydriden von Phosphor-, Phosphon- und Phosphinsäuren bei einer Reaktionstemperatur zwischen -10°C und dem Siedepunkt des Lösungsmittels, vorzugsweise zwischen -5°C und 40°C durchgeführt werden.

Geeignete Lösungsmittel dafür sind Dimethylformamid, Dimethylacetamid, N-Methylpyrrolidon oder Dimethylsulfoxid. Sofern die Löslichkeit der Komponenten es erlaubt, können auch Lösungsmittel wie Methylenchlorid, Chloroform oder Tetrahydrofuran oder Gemische der genannten Lösungsmittel eingesetzt werden. Die genannten Methoden sind z.B. in Meinhofer-Gross: "The Peptides" Academic Press, Vol. I, (1979) beschrieben.

Falls zur Verhinderung von Nebenreaktionen oder für die Synthese spezieller Peptide erforderlich, sind die

funktionellen Gruppen in der Seitenkette von Aminosäuren durch geeignete Schutzgruppen (siehe z.B. T.W. Greene, "Protective Groups in Organic Synthesis") zusätzlich geschützt, wobei in erster Linie Arg$(BOC)_2$, Arg(Tos), Arg(Mts), Arg(Mtr), Arg(PMV), Asp(OBzl), Asp(OBut), Cys(4-MeBzl), Cys(Acm), Cys(SBut), Glu(OBzl), Glu(OBut), His(Tos), His (Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Tyr(Br-Z), Tyr(Bzl) oder Tyr(But) eingesetzt werden.

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest und der durch sekundäre Amine abspaltbare 9-Fluorenylmethyloxycarbonyl- (Fmoc)-Rest herangezogen. Die SH-Gruppe des Cysteins kann durch eine Reihe von Schutzgruppen blockiert sein. Bevorzugt wird hier der Trityl-(Trt)-Rest und der S-tert.-Butyl(StBu-)Rest. Der Tritylrest kann durch Jod-Oxydation unter Bildung der Cystin-Verbindungen oder durch reduzierende saure Spaltung zu den Cystein-Verbindungen abgespalten werden (Liebigs Ann. Chem. 1979, 227-247).

Der S-tert.-Butyl-Rest wird dagegen am besten mit Tributylphosphin reduktiv gespalten (Aust. J. Chem. 19 (1966) 2355-2360). OH- und COOH-Funktionen in den Seitenketten werden am besten durch den sauer abspaltbaren tert.-Butyl-(tBu-)Rest geschützt (siehe auch: Meienhofer-Gross: "The Peptides", Vol. 3).

Die Verbindungen der Formeln VI bzw. VII werden wie folgt hergestellt:

**Schema 1**

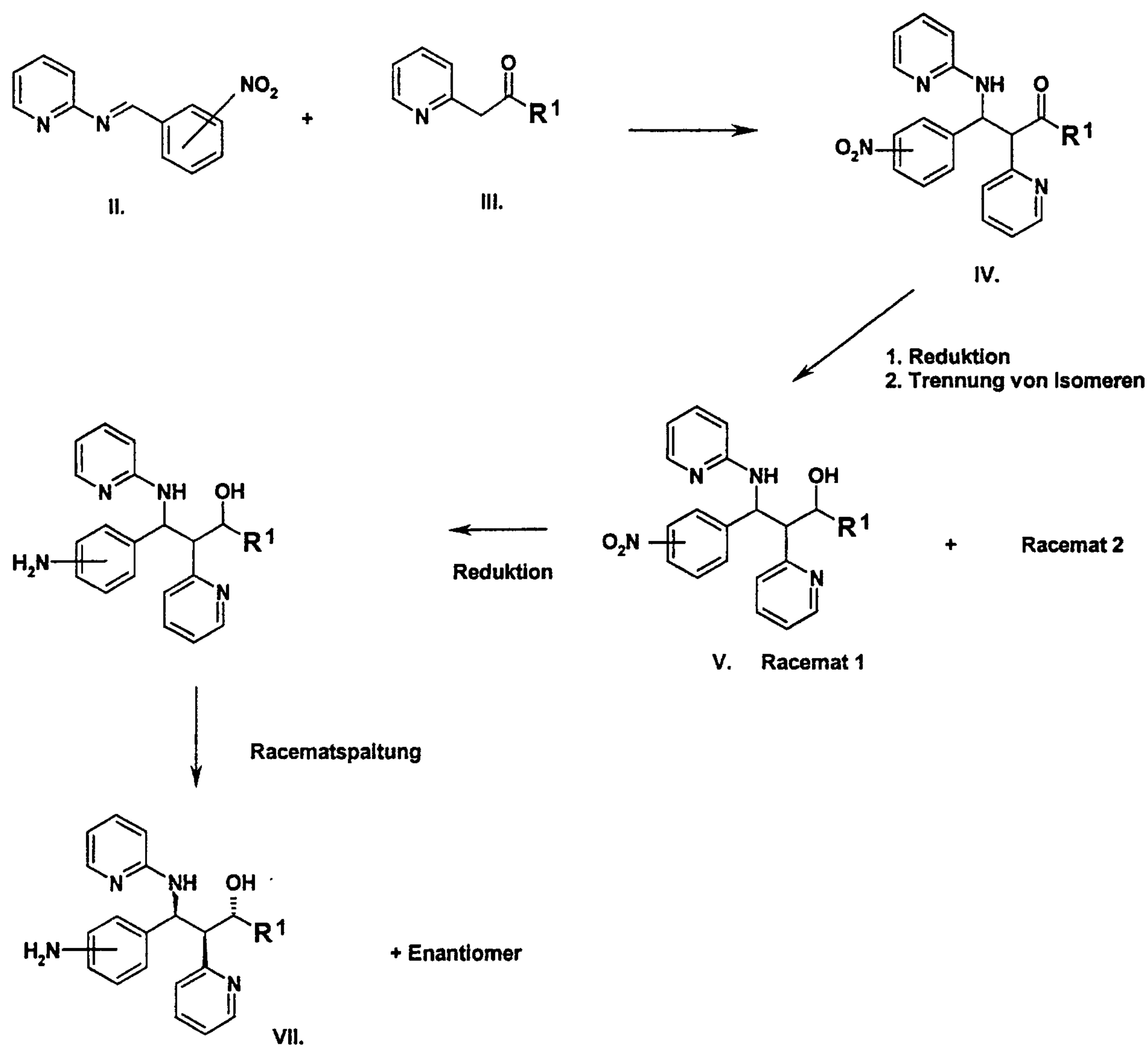

Verbindungen des Typs IV werden erhalten, indem o-, m- oder p-substituierte Imine des Typs II mit dem Keton III zur Reaktion gebracht werden. Die Reaktion kann zum Beispiel durch Mischung der beiden Verbindungen in Substanz, ohne Lösungsmittel, und anschließendem Erhitzen oder in einem geeigneten Lösungsmittel wie Ethanol, Tetrahydrofuran (THF), Toluol, Diglyme oder Tetradecan bei Temperaturen von 20° C bis 150° C durchgeführt werden.

Die Ketoverbindungen des Typs IV werden in einem geeigneten Lösungsmittel, wie z.B. Methanol, THF oder THF/Wasser mit $NaBH_4$ oder einem anderen geeigneten Reduktionsmittel bei Temperaturen zwischen -30° C und + 40° C zu Hydroxyverbindungen des Typs V reduziert. Bei der Reduktion fallen meist zwei Isomerengemische (Racemate) als Hauptprodukte an. Die unterschiedlichen Racemate können durch fraktionierte Kristallisation oder durch Kieselgelchromatographie voneinander abgetrennt werden. Die Nitrogruppe in Verbindungen des Typs V kann durch bekannte Verfahren, wie z.B. die katalytische Hydrierung mit Pd oder Pd auf Kohle und $H_2$ in Methanol, reduziert werden.

Die so erhaltenen racemischen Verbindungen des Typs VI kann weiter in ihre Enantiomere aufgetrennt werden. Die Racematspaltung von VI in Enantiomere des Typs VII kann durch Chromatographie über chirales Säulenmaterial oder durch literaturbekannte Verfahren mit optisch aktiven Hilfsreagenzien durchgeführt werden (vgl. J. Org. Chem. **44,** 1979, 4891).

Herstellung der erfindungsgemäßen Verbindungen der Formel I ausgehend von Verbindungen des Typs VI oder VII.

Verfahren A

Schema 2

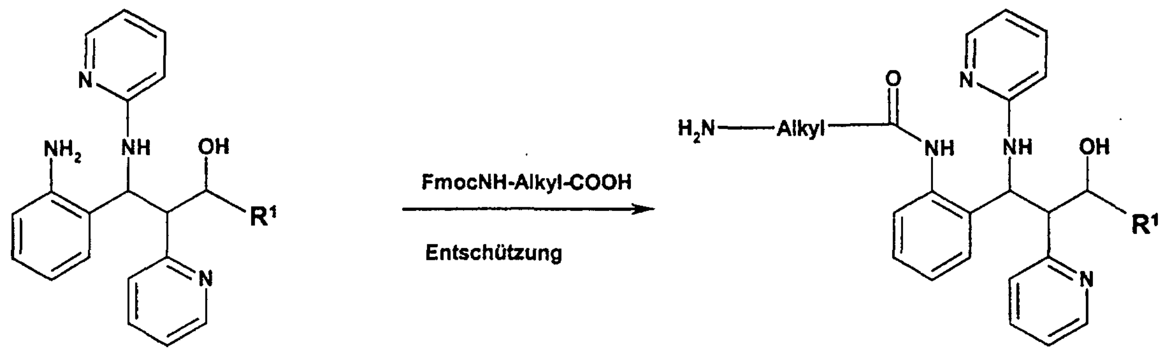


VI oder VII

VIII

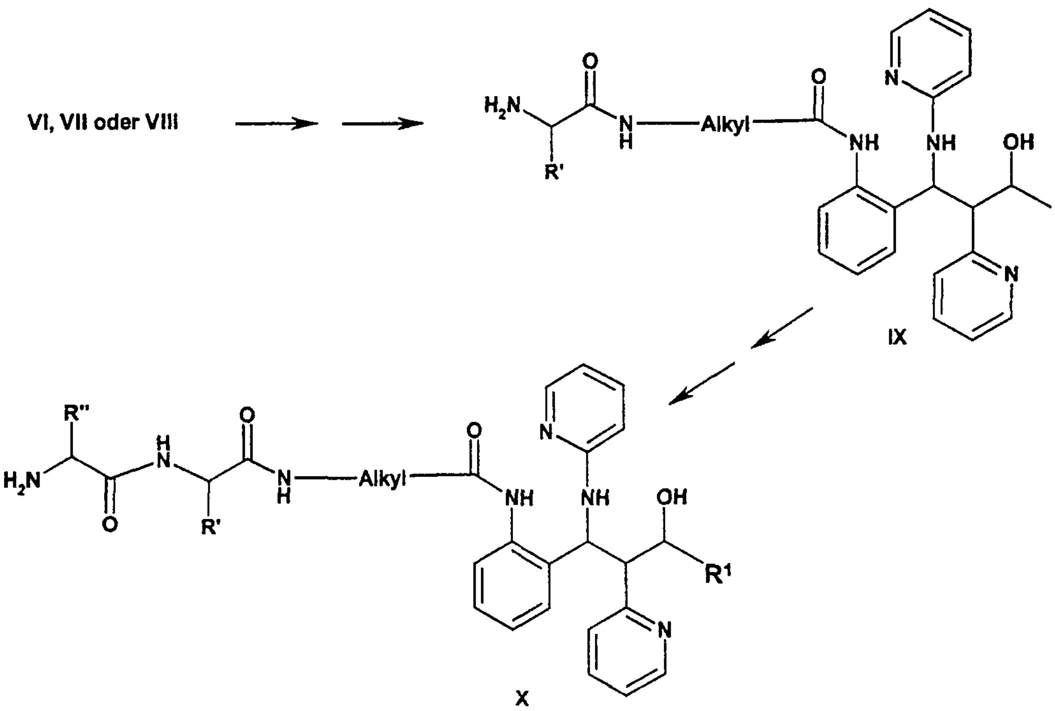


IX

X

Verbindungen der Formel VI oder VII werden mit Derivaten von Aminoalkancarbonsäuren zur Reaktion gebracht. Hierbei werden Peptidkupplungsverfahren eingesetzt. Die Aminoalkancarbonsäuren, z.B. Glycin, β-Alanin oder ω-Aminoundecansäure sind mit Fmoc-Gruppen geschützt, es können z.B. auch entsprechende Nitro- oder Azidocarbonsäuren verwendet werden. Nach Abspaltung der Schutzgruppe in einem zweiten Schritt, oder entsprechend nach Reduktion der Azido- oder Nitrogruppe werden Verbindungen der Formel VIII erhalten. Verbindungen der Formeln VI, VII oder VIII können mit aminogeschützten, z.B. Fmoc-geschützten Aminosäuren nach Peptidkupplungsverfahren umgesetzt werden. die Seitenketten können mit geeigneten orthogonalen Schutzgruppen geschützt oder ungeschützt sein. Nach der Kupplungsreaktion wird die Schutzgruppe der Aminofunktion abgespalten, im Fall von Fmoc z.B. mit

Piperidin in DMF. Die daraus erhaltenen Verbindungen des Typs IX können in ein bis drei weiteren Reaktionssequenzen - Aminosäurekupplung, Abspaltung der Aminoschutzgruppe - zu Verbindungen der Formel X umgesetzt werden. Die Schutzgruppen der Seitenketten der bis zu vier Aminosäuren $A^1$ bis $A^4$ können nach jeder Reaktionssequenz einzeln, nach allen Kupplungsreaktionen gemeinsam abgespalten werden oder auch alle oder teilweise auf den erfindungsgemäßen Verbindungen X verbleiben.

Verfahren B

Schema 3

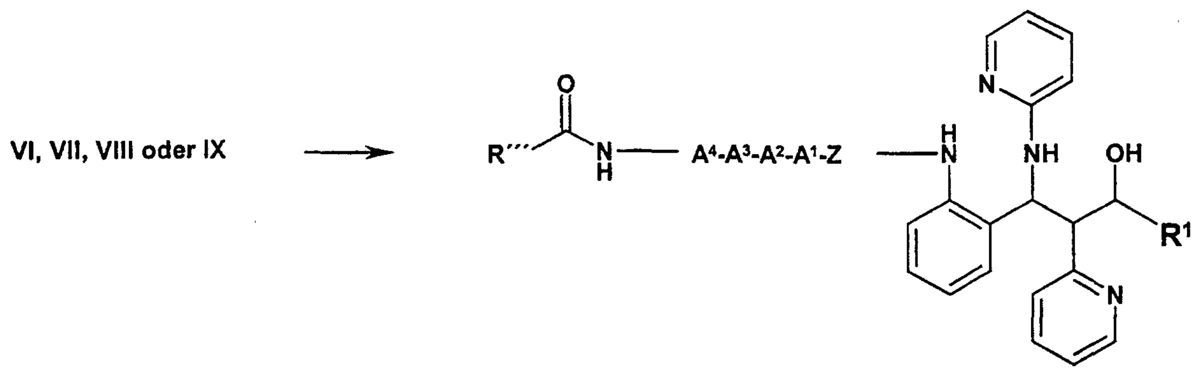


XI

Die freien Aminofunktionen von Verbindungen der Formeln VI, VII, VIII, IX oder X werden mit Carbonsäuren, ebenfalls nach üblichen Amidbildungsmethoden, zur Reaktion gebracht. Funktionelle Gruppen der Ausgangsverbindungen, die zu Nebenreaktionen führen können, müssen in geschützter Form vorliegen und können, falls erforderlich, nach der Reaktion mit der Carbonsäure abgespalten werden. Daraus werden die erfindungsgemäßen Verbindungen des Typs XI erhalten.

Verfahren C

Schema 4

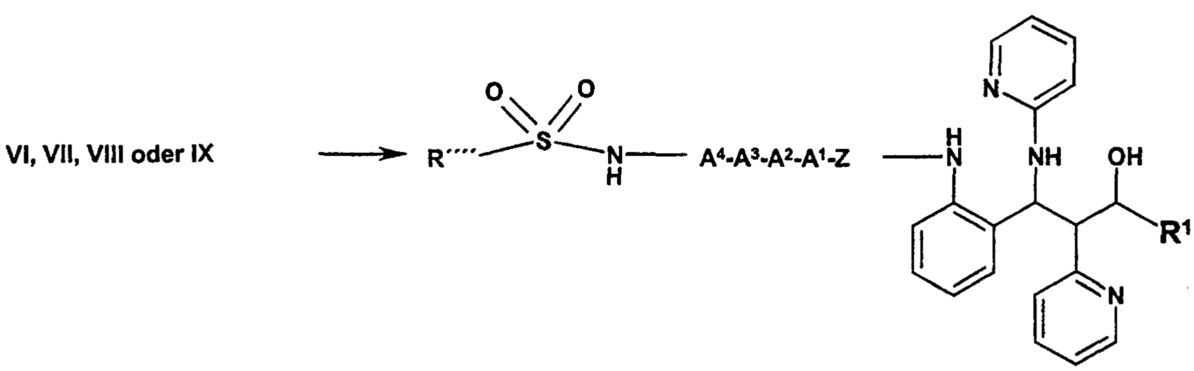


XI

Analog zum Verfahren B werden aus den Verbindungen der Formeln VI bis IX die Sulfonamidderivate XII erhalten. Zur Herstellung können die Aminofunktionen der Ausgangsverbindungen z.B. mit Sulfonsäurechloriden in

Gegenwart einer Hilfsbase in einem geeigneten Lösungsmittel zur Reaktion gebracht werden.

Verfahren D

Schema 5

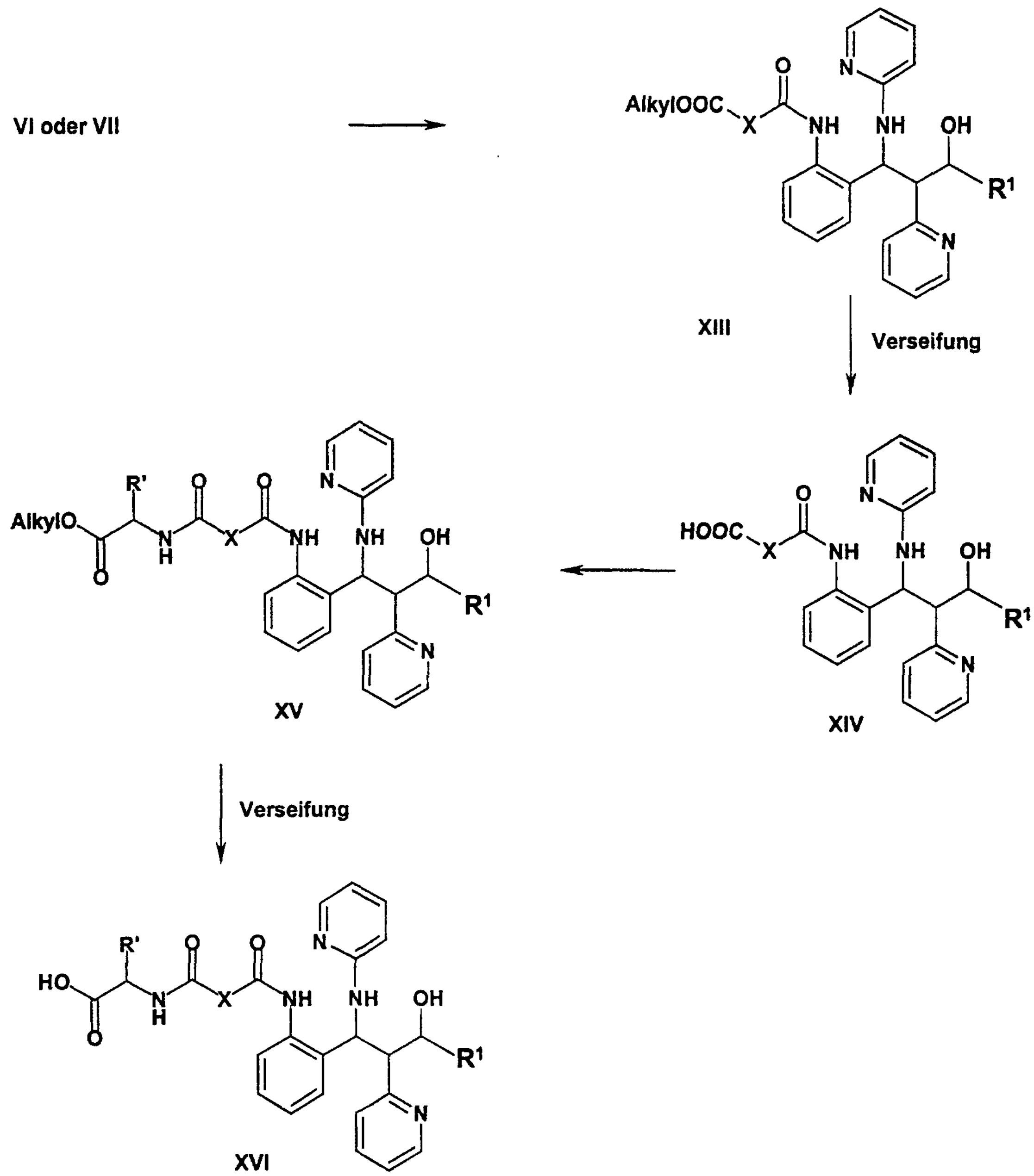


Verbindungen des Typs XIII können durch Reaktion von Dicarbonsäuremonoalkylestern mit Verbindungen des Typs VI oder VII erhalten werden, wobei X entsprechend den Ansprüchen einen Alkyl- oder einen Phenylrest darstellt. Die Reaktion wird mit üblichen Peptidkupplungsverfahren durchgeführt. Anschließend wird die Alkylesterfunktion zur Carbonsäure verseift, um Verbindungen der Formel XIV zu erhalten. Die Verbindungen XIV können auch direkt aus den Aminen des Typs VI oder VII durch Reaktion mit Dicarbonsäureanhydriden, z.B. Bernsteinsäureanhydrid, in Gegenwart einer Base erhalten werden. Wird die Carbonsäurefunktion den Verbindungen XIV mit Aminosäurealkylestern, die in der Seitenkette eine Schutzgruppe tragen könne, umgesetzt, werden Verbindungen der Formel XV

erhalten. Hieraus werden wiederum durch Verseifung der Alkylesterfunktion die Verbindungen der Formel XVI hergestellt.
Sinngemäß könne die Verfahren A - D auch so modifiziert werden, daß die erfindungsgemäßen Verbindungen durch Reaktionen an fester Phase hergestellt werden könne. Dies wird im Verfahren E an einem allgemeinen Beispiel dargestellt.

Verfahren E

Schema 6

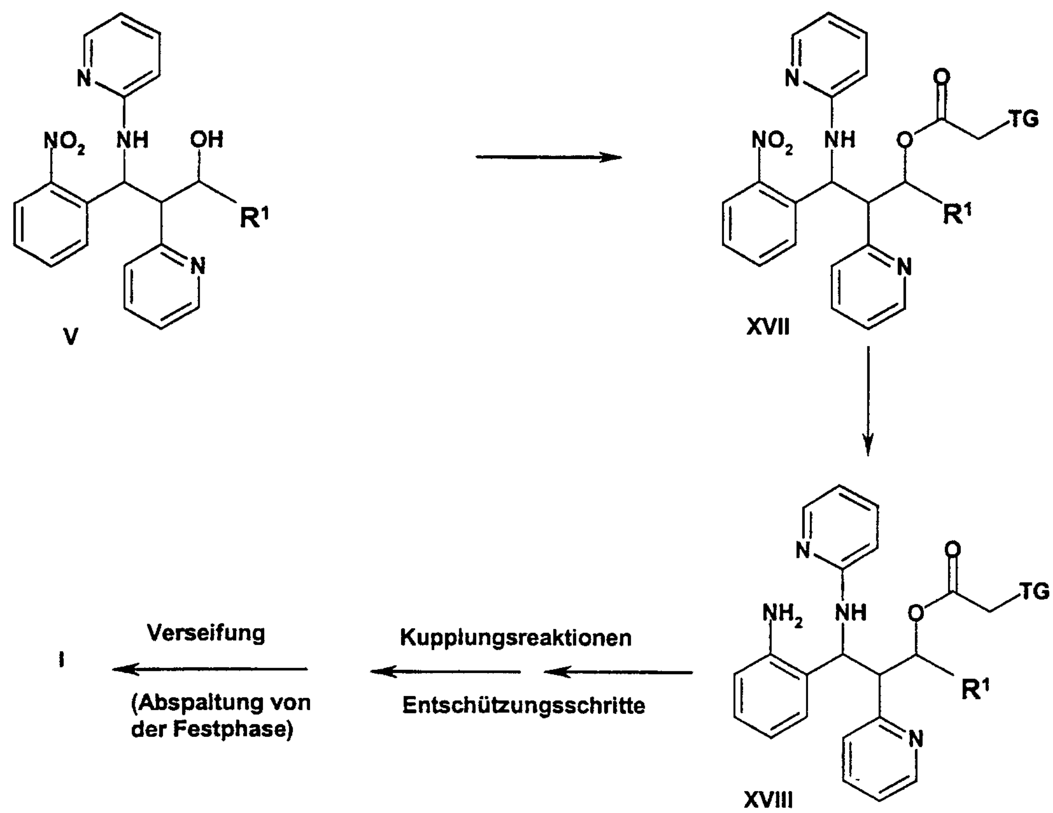


Die Verbindung der Formel V wird auf ein modifiziertes Polystyrolharz gekuppelt. Hierbei wird die Carboxylgruppe von Carboxy-Tentagel (Fa. Rapp, Tübingen) mit der OH-Funktion der Verbindung VI mit Veresterungsmethoden, z.B. DCC, DMAP zur Reaktion gebracht. Die Nitrogruppe der so erhaltenen Verbindung XVII wird mit geeigneten Methoden, z.B. $SuCl_2$-Reduktionsverfahren, in die Aminofunktion überführt. Am festphasengebundenen Derivat XVIII wird nun in Analogie zu den bereits beschriebenen Peptidkupplungsverfahren die Seitenkette $(E)_7$-$(A^4)_p$-$(A^3)_o$-$(A^2)_n$-$(A^1)_m$-$(Z)_e$ bis zur gewünschten Länge aufgebaut. Im letzten Schritt werden die erfindungsgemäßen Verbindungen der Formel I durch Verseifung der Estergruppe unter basischen Bedingungen von der festen Phase abgespalten.

Die in den beschriebenen Verfahren als Schutzgruppen der Aminosäureseitenketten bezeichneten Reste können in den erfindungsgemäßen Verbindungen verbleiben oder aber nach bekannten Methoden (siehe T.W. Green "Protective Groups in Organic Synthesis") abgespalten werden.

Die so erhaltenen Verbindungen der Formel I können gegebenenfalls in ihr pharmazeutisch verträgliches Salz oder physiologisch funktionelles Derivat überführt werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).
Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Benzothiazepin-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung sind weiterhin sowohl Isomerengemische der Formel I, als auch die reinen Enantiomere der Formel I.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen. Folgende Befunde belegen die pharmakologische Wirksamkeit der erfindungsgemäßen Verbindungen.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml T61®, einer wäßrigen Lösung von 2,5 mg Tetracain HCl, 100 m Embutramid und 25 mg Mebezoniumjodid getötet. Der Dünndarm wurde entnommen und mit eiskalter physiologischer Kochsalzlösung gespült. Die terminalen 7/10 des Dünndarms (gemessen in oral-rektaler Richtung, d.h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80°C eingefroren. Zur Präparation der Membranvesikel wurden die eingefrorenen Därme bei 30°C im Wasserbad aufgetaut. Die Mucosa wurde abgeschabt und in 60 ml eiskaltem 12 mM Tris/HCl-Puffer (pH 7,1)/300 mM Mannit, 5 mM EGTA/ 10 mg/l Phenylmethylsulfonylfluorid/1 mg/l Trypsin Inhibitor v. Sojabohnen (32 U/mg)/0,5 mg/l Trypsin Inhibitor v. Rinderlunge (193 U/mg)/5 mg/l Bacitracin suspendiert. Nach dem Verdünnen auf 300 ml mit eiskaltem destilliertem Wasser wurde mit einem Ultraturrax (18-Stab, IKA Werk Staufen, Deutschland) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0°C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wurde der Niederschlag verworfen und der Überstand, der die Bürstensaummembranen enthält, 30 Minuten bei 48000 x g (20000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-mi-

nütiger Inkubationszeit bei 0°C wurde erneut 15 Minuten bei 3000 x g zentrifugiert. Der Überstand wurde anschließend nochmals 30 Minuten bei 48000 x g (20000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196°C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol) /0,5 µl 10 mM Taurocholat/8,75 µl Natrium-Transport-Puffer (10 mM Tris/Hepes (pH 7,4)/100 mM Mannit/100 mM NaCl) (Na-T-P) bzw. 8,75 µl Kalium-Transport-Puffer (10 mM Tris/Hepes (pH 7,4)/ 100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, Deutschland) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes (pH 7,4)/150 mM KCl) gestoppt. Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, Deutschland) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, Deutschland) aufgelöst und die Radioaktivität durch Flüssigkeits-Szintillationsmessung in einem Meßgerät TriCarb 2500 (Canberra Packard GmbH, Frankfurt, Deutschland) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 1 zusammengefaßt.

Tabelle 1 zeigt Meßwerte der Hemmung der [$^3$H]-Taurocholataufnahme in Bürsensaummembranvesikel des Ileums von Kaninchen. Angegeben sind die Quotienten aus den $IC_{50Na}$-Werten der Referenzsubstanz als Taurochenodesoxycholat (TCDC) und der jeweiligen Testsubstanz.

Tabelle 1:

| Verbindungen aus Beispiel | $\frac{IC_{50Na}\text{-TCDC [µmol]}}{IC_{50Na}\text{-Substanz [µmol]}}$ |
|---|---|
| 2c | 1.06 |
| 3 | 0.88 |
| 6 | 0.77 |
| 7 | 0.87 |
| 14 | 0.21 |
| 15 | 0.94 |
| 16 | 0.16 |
| 17 | 1.26 |
| 18 | 0.69 |

Tabelle 1: (fortgesetzt)

| Verbindungen aus Beispiel | $\frac{IC_{50Na}\text{-TCDC } [\mu mol]}{IC_{50Na}\text{-Substanz } [\mu mol]}$ |
|---|---|
| 19 | 1.05 |
| 20 | 0.30 |
| 21 | 0.17 |
| 22 | 0.82 |
| 31 | 1.13 |
| 33 | 0.52 |
| 34 | 0.81 |
| 35 | 0.36 |
| 36 | 0.36 |
| 38 | 0.38 |
| 41 | 0.61 |
| 44 | 1.05 |
| 45 | 1.03 |
| 47 | 1.00 |
| 49 | 0.86 |
| 50 | 0.67 |
| 52 | 1.11 |
| 53 | 0.46 |
| 56 | 1.15 |
| 57 | 0.79 |
| 60 | 0.62 |
| 61 | 0.66 |
| 62 | 0.99 |
| 64 | 0.39 |
| 65 | 0.84 |
| 66 | 0.93 |
| 69 | 1.00 |
| 73 | 0.92 |
| 74 | 0.70 |
| 77 | 0.22 |
| 78 | 0.27 |
| 82 | 0.79 |
| 83 | 0.24 |
| 87 | 0.84 |
| 89 | 0.90 |
| 91 | 0.92 |
| 93 | 1.10 |

Tabelle 1: (fortgesetzt)

| Verbindungen aus Beispiel | $\frac{IC_{50Na}\text{-TCDC [µmol]}}{IC_{50Na}\text{-Substanz [µmol]}}$ |
|---|---|
| 94 | 0.40 |
| 143 | 0.26 |
| 144 | 1.16 |
| 145 | 1.19 |
| 146 | 0.87 |
| 148 | 0.36 |
| 149 | 0.34 |
| 132 | 0.82 |
| 117 | 0.78 |
| 120 | 0.76 |

Die nachfolgenden Beispiele dienen zur näheren Erläuterung der Erfindung, ohne dieselbe auf in den Beispielen beschriebene Produkte und Ausführungsformen einzuschränken.

Beispiel 1

a.

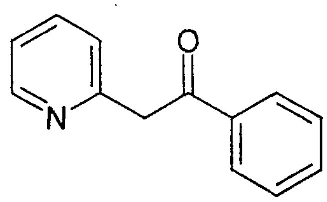

Zu 50 g (0.54 mol) Picolin in 770 ml Tetrahydrofuran wurden bei -55°C 366 ml 15 % n-Butyllithium in n-Hexan zugetropft. Es wurde auf Zimmertemperatur erwärmt und erneut auf -55°C gekühlt. 77 g N,N-Dimethylbenzamid (0.52 mol) in 570 ml Tetrahydrofuran wurden langsam zugetropft, anschließend auf Zimmertemperatur erwärmt und noch 1 h gerührt. Nach der Zugabe von 550 ml 1 N Salzsäure wurde mit Ethylacetat extrahiert (3x), die organischen Phasen mit $MgSO_4$ getrocknet und eingedampft. Durch Destillation des Rückstandes wurden 47.5 g (47%) Produkt erhalten. Siedepunkt 134-136°C/0.28 mbar.

b.

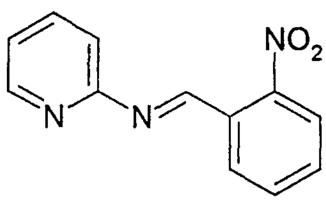

20.0 g (0.13 mol) σ-Nitrobenzaldehyd, 12.5 g (0.13 mol) 2-Aminopyridin und 0.3 g p-Toluolsulfonsäure wurden in 150 ml Toluol 2.5 h am Wasserabscheider unter Rückfluß erhitzt. Die Lösung wurde abgekühlt, der entstandene Niederschlag abgesaugt und getrocknet.

Ausbeute: 18.1 g (60%) Produkt
Schmelzpunkt: 93-95°C

$C_{12}H_9N_3O_2$ (227) MS (FAB) 228 M + $H^+$

b.

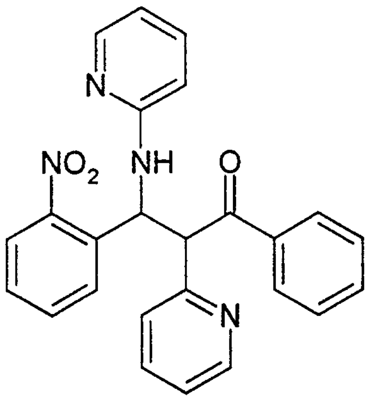

12.0 g (61 mmol) Keton aus Beispiel 1 a und 15.0 g (66 mmol) Imin aus Beispiel 1 b wurden 45 min auf dem Dampfbad erwärmt. Das Reaktionsgemisch wurde in Ethanol unter Erwärmen gelöst. Nach dem Abkühlen wurde der Niederschlag abgesaugt und aus Ethanol umkristallisiert.

Ausbeute: 11.8 g (46%) Produkt

$C_{25}H_{20}N_4O_3$ (424.2) MS (FAB) 425 M + $H^+$

d.

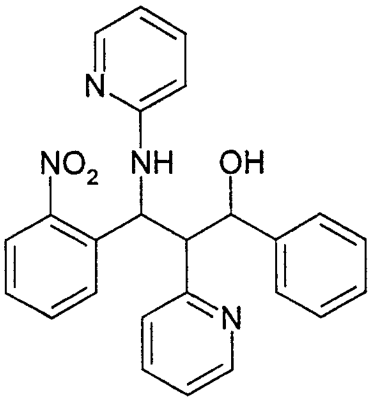

8.0 g (18.8 mmol) Ketoverbindung aus Beispiel 1 c wurden in 300 ml Tetrahydrofuran/Wasser 10:1 gelöst, mit 4.67 g Natriumborhydrid versetzt und 2 h bei Zimmertemperatur gerührt. Anschließend wurde die Lösung eingedampft, der Rückstand mit 100 ml 2N Salzsäure versetzt und auf dem Dampfbad erwärmt bis alles gelöst war. Nach dem Abkühlen wurde mit 4N NaOH-Lösung basisch gestellt und mit Ethylacetat (2x) extrahiert. Die organischen Phasen wurden mit $MgSO_4$ getrocknet und eingedampft. Der Rückstand wurde über Kieselgel chromatographiert (Heptan/Ethylacetat 1:1). Es wurden zwei racemische Verbindungen als Produkt erhalten.

1. Fraktion: 3.9 g (48 %) unpolares Racemat (Bsp. 1 d/1)
$C_{25}H_{22}N_4O_3$ (426.2) MS (FAB) 427 M + $H^+$
2. Fraktion: 2.5 g (31 %) polares Racemat (Bsp. 1 d/2)
$C_{25}H_{22}N_4O_3$ (426.2) MS (FAB) 427 M + $H^+$

e.

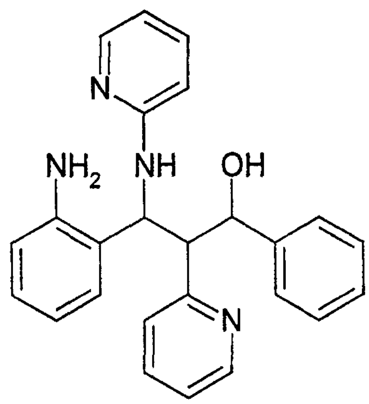

2.5 g (5.86 mmol) des unpolaren Racemats aus Beispiel 1 d/1 wurden in 300 ml Methanol gelöst, mit ca. 20 mg Pd/C 10 % versetzt und unter $H_2$-Atmosphäre bei Zimmertemperatur hydriert. Vom Katalysator wurde abfiltriert und die Lösung eingedampft. Der Rückstand wurde über Kieselgel chromatographiert (n-Heptan/Ethylacetat 7:13).

| | |
|---|---|
| Ausbeute: | 1.9 g (82%) Produkt |
| $C_{25}H_{24}N_4O$ | (396.22) MS (FAB) 397 M + $H^+$ |

f.

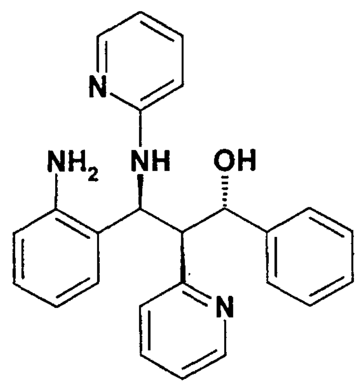

**(+)-Enantiomer**

**(Beispiel 1 f/2)**

100 mg der racematischen Verbindung aus Beispiel 1 e wurde durch präparative HPLC in die Enantiomere getrennt. Die Trennung erfolgte über eine CSP-Chiralpak-Säule (Fa. Daicel, Düsseldorf) mit n-Hexan/Ethanol 4:1. Als erste Fraktion wurden 40 mg des (-)-Enantiomers (Bsp. 1 f/1) als 2. Fraktion 40 mg des (+)-Enantiomers (Bsp. 1 f/2) erhalten.

g.

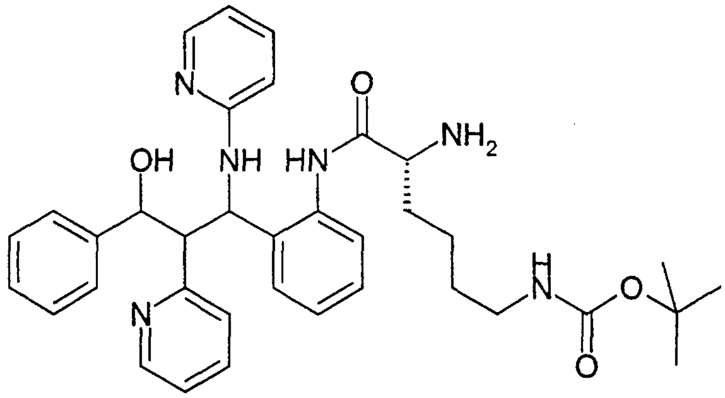

4.0 g (10.1 mmol) der Aminoverbindung aus Beispiel 1e (unpolares Racemat), 4.85 g (10.3 mmol) N-Fmoc-D-Lys(BOC)OH, 4.0 g (12.2 mmol) TOTU und 2.7 ml Triethylamin wurden in 300 ml Dimethylformamid gelöst und 2 h bei Zimmertemperatur gerührt. Das Reaktionsgemisch wurde auf Wasser gegossen und mit Ehylacetat (2 x) extrahiert. Die organischen Phasen wurden getrocknet ($MgSO_4$) und eingedampft. Der Rückstand wurde in 150 ml Dimethylformamid/Piperidin 2:1 zur Abspaltung der Fmoc-Gruppe gelöst und 1 h bei Zimmertemperatur gerührt. Es wurde auf Wasser gegossen, mit Ethylacetat (3 x) extrahiert. Die organischen Phasen wurden getrocknet ($MgSO_4$) und eingedampft. Chromatographie über Kieselgel (Dichlormethan/Methanol 9:1) ergab 4.0 g (63.5 %) Produkt

$C_{36}H_{44}N_6O_4$ (624.3) MS (FAB) 625 $M + H^+$

h.

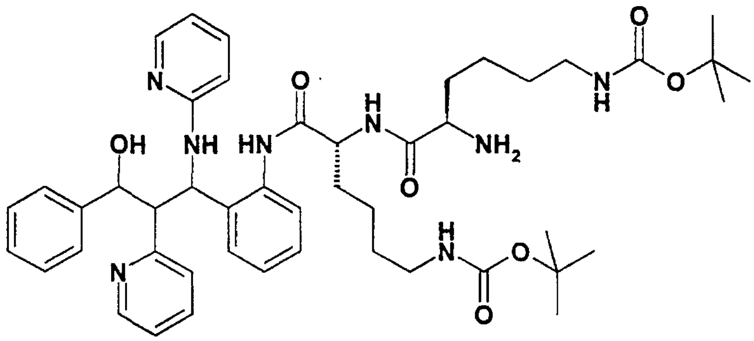

Aus 8,0 g der Verbindung aus Beispiel 1 g (12.8 mmol) und 6.4 g (13.7 mmol) N-Fmoc-D-Lys(BOC)OH wurden nach dem unter 1 g beschriebenen Verfahren 4.74 g (43%) Produkt erhalten.

$C_{47}H_{64}N_8O_7$ (852.5) MS (FAB) 853.5 $M + H^+$

Beispiel 2

a.

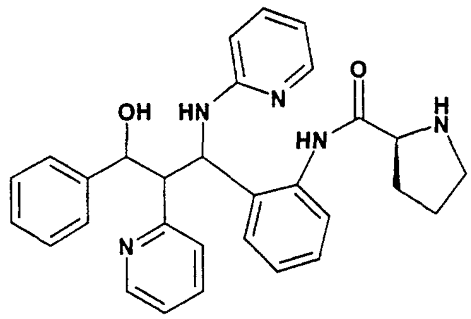

2.5 g (6.31 mmol) Aminoverbindung aus Beispiel 1 e (unpolares Racemat), 2.2 g (6.52 mmol) Fmoc-L-Prolin, 2.5 g (7.62 mmol) TOTU und 1.7 ml Triethylamin wurden in 100 ml Dimethylformamid gelöst und 3 h bei Zimmertemperatur gerührt. Das Reaktionsgemisch wurde auf die Hälfte eingedampft, mit Wasser versetzt und mit Ethylacetat extrahiert (3 x). Die organischen Phasen wurden über $MgSO_4$ getrocknet und eingedampft. Nach Chromatographie über Kieselgel (Ethylacetat/n-Heptan 7:3) wurden 3.85 g (85 %) Produkt erhalten.

Dieses Fmoc-geschützte Zwischenprodukt (3.6 g) wurde in 110 ml Piperidin/DMF 1:10 gelöst und 1 h bei Zimmertemperatur gerührt. Das Gemisch wurde eingedampft und über Kieselgel (Dichlormethan/Methanol 19:1, dann 9:1) chromatographiert.

Ausbeute: 1.8 g (72.5 %)

$C_{30}H_{31}N_5O_2$ (493.2) MS (FAB) 494 M + $H^+$

b.

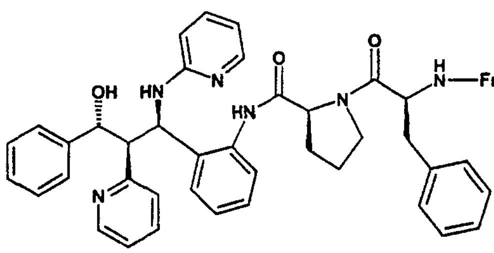

Bsp. 2 b/1

and

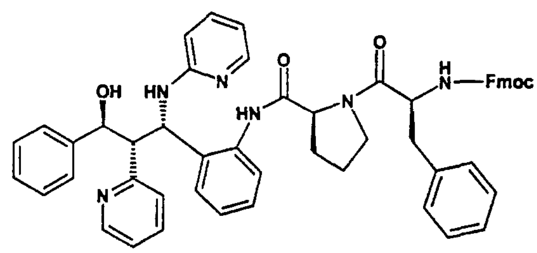

Bsp. 2 b/2

1.7 g (3.44 mmol) der Verbindung aus Beispiel 2 a wurden mit 1.4 g (3.61 mmol) Fmoc-L-Phenylalanin, 1.9 g (5.80 mmol) TOTU und 1.0 ml Triethylamin in 150 ml DMF 4 h bei Zimmertemperatur gerührt. Das Reaktionsgemisch wurde eingedampft und der Rückstand über Kieselgel chromatographiert (Ethylacetat/n-Heptan 4:1). Es wurden zwei Fraktionen erhalten:

1. Fraktion 1.28 g (43 %) unpolares Diastereomer (Bsp. 2 b/1)
$C_{54}H_{50}N_6O_5$ (862.4) MS (FAB) 863.4 M + $H^+$
2. Fraktion 0.82 g (28 %) polares Diastereomer (Bsp. 2 b/1)
$C_{54}H_{50}N_6O_5$(862.4) MS(FAB) 863.4 M + $H^+$

C.

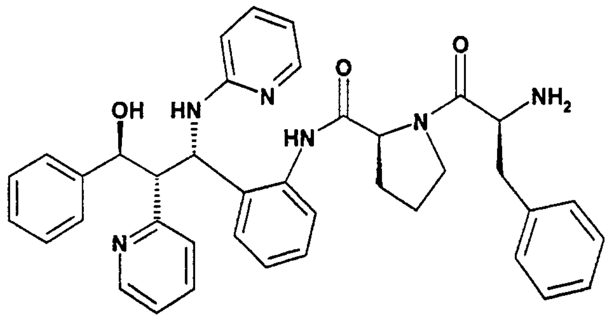

0.8 g (0.93 mmol) Verbindung aus Beispiel 2 b/2 wurden in 33 ml DMF/Piperidin 10:1 gelöst und1 h bei Zimmertemperatur gerührt. Nach dem Eindampfen wurde der Rückstand über Kieselgel chromatographiert (Dichlormethan/Methanol 19:1, dann 9:1).

Ausbeute: 0.35 g (59 %).

$C_{39}H_{40}N_6O_3$ (640.3) MS (FAB) 641.3 M + $H^+$

In Analogie zu den Beispielen 1g und 2 a wurden ausgehend von den Beispielen 1e und 1f die Beispiele der Tabelle 2 erhalten.

Tabelle 2:

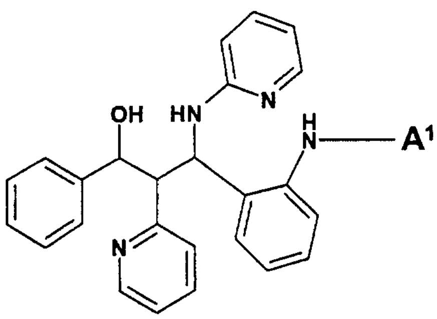

| Beispiel | Aminosäurerest $A^1$ | Summenformel (Molmasse) | MS (FAB) |
|---|---|---|---|
| 3 | Gly | C27 H27 N5 O2 (453.5) | 454.5 M+$H^+$ |
| 4 | L-Tyr(t-But) | C38 H41 N5 O3 (615.7) | 616.7 M+$H^+$ |

| Beispiel | Aminosäurerest $A^1$ | Summenformel (Molmasse) | MS (FAB) |
|---|---|---|---|
| 5 | L-Ser(t-But) | C32 H37 N5 O3..(539.6) | 540.6 $M+H^+$ |
| 6 | L-Lys(BOC) | C36 H44 N6 O4..(624.7) | 625.7 $M+H^+$ |
| 7 | L-Tyr | C34 H33 N5 O3..(559.6) | 560.6 $M+H^+$ |
| 8 | L-Ser | C28 H29 N5 O3..(483.6) | 484.6 $M+H^+$ |
| 9 | L-Lys | C31 H36 N6 O2..(524.7) | 525.7 $M+H^+$ |
| 10 | L-Arg$(BOC)_2$ | C41 H52 N8 O6..(752.9) | 753.9 $M+H^+$ |
| 11 | L-Ornithin(BOC) | C35 H42 N6 O4..(610.7) | 611.7 $M+H^+$ |
| 12 | 2,4-Diaminobutter-säure(BOC) | C34 H40 N6 O4..(596.7) | 597.7 $M+H^+$ |

In Analogie zu den Beispielen 1h und 2c wurden ausgehend von den Beispielen 1e und 1f die Beispiele der Tabelle 3 erhalten.

Tabelle 3:

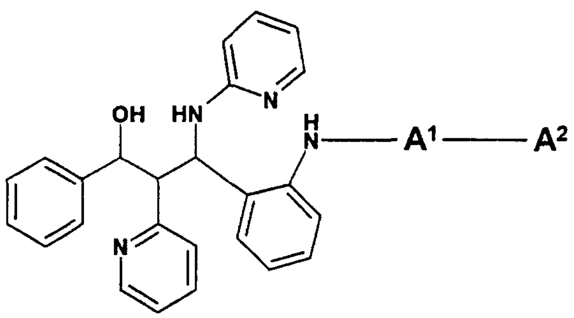

| Beispiel | Aminosäurerest $A^1$ | Aminosäurerest $A^2$ | Summenformel (Molmasse) | MS (FAB) |
|---|---|---|---|---|
| 13 (polar) | Gly | Gly | C29 H30 N6 O3 (510.6) | 511.6 $M+H^+$ |
| 14 (unpolar) | L-Lys(BOC) | L-Lys(BOC) | C47 H64 N8 O7 (853.1) | 854.1 $M+H^+$ |
| 15 (polar) | L-Lys(BOC) | L-Lys(BOC) | C47 H64 N8 O7 (853.1) | 854.1 $M+H^+$ |
| 16 (unpolar) | L-Lys(BOC) | L-Ser(BOC) | C43 H57 N7 O6 (760.0) | 761.0 $M+H^+$ |
| 17 | L-Lys(BOC) | L-Ser(BOC) | C43 H57 N7 O6 | 761.0 $M+H^+$ |

| | | | | |
|---|---|---|---|---|
| (polar) | | | (760.0) | |
| 18 (unpolar) | L-Lys(BOC) | L-Arg | C42 H56 N10 O5 (781.0) | 782.0 $M+H^+$ |
| 19 (polar) | L-Lys(BOC) | L-Arg | C42 H56 N10 O5 (781.0) | 782.0 $M+H^+$ |
| 20 | L-Phe | L-Ser(BOC) | C41 H46 N6 O (686.8) | 687.8 $M+H^+$ |
| 21 | L-Phe | L-Phe | C43 H42 N6 O3 (690.8) | 691.8 $M+H^+$ |
| 22 | L-Phe | L-Lys(BOC) | C45 H53 N7 O5 (772.0) | 773.0 $M+H^+$ |

Tabelle 4

| Beispiel | Formel | Summenformel | Molmasse | MS (FAB) |
|---|---|---|---|---|
| 30 | Chiral 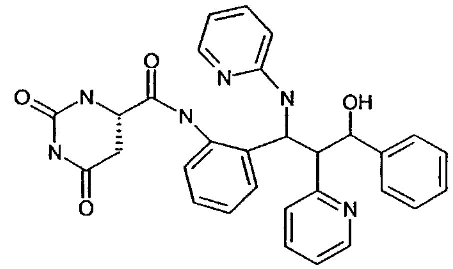 | C30 H28 N6 O4 | 536,6 | 537.6 M+H$^+$ |
| 31 | 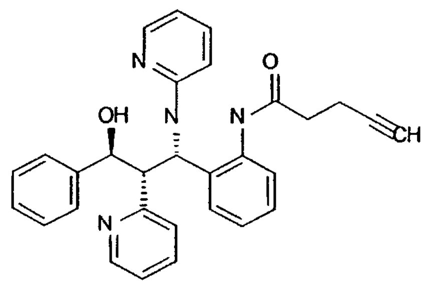 | C30 H28 N4 O2 | 476,6 | 477.6 M+H$^+$ |
| 32 | 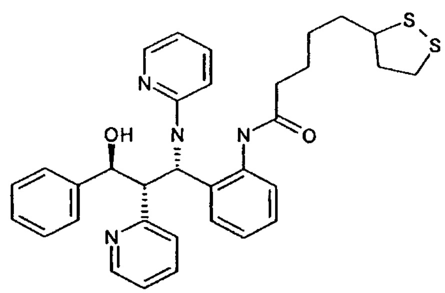 | C33 H36 N4 O2 S2 | 584,8 | 585,8 M+H$^+$ |

| | | | | |
|---|---|---|---|---|
| 33 | 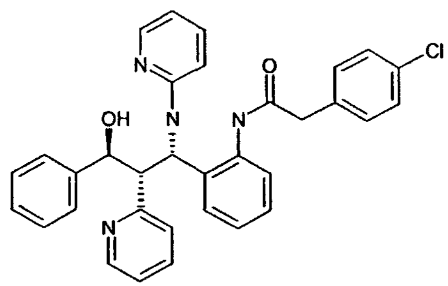 | C33 H29 Cl N4 O2 | 549,1 | 550.1 M+H+ |
| 34 | 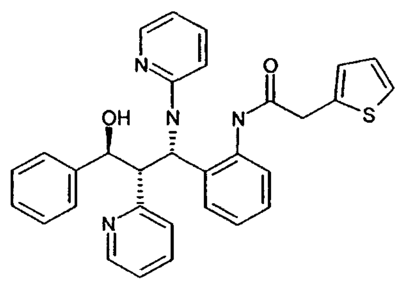 | C31 H28 N4 O2 S | 520,7 | 521.7 M+H+ |
| 35 | 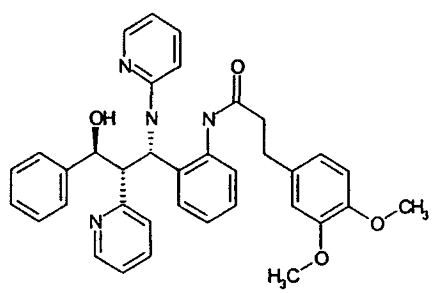 | C36 H36 N4 O4 | 588,7 | 589.7 M+H+ |

| | | | | |
|---|---|---|---|---|
| 36 | 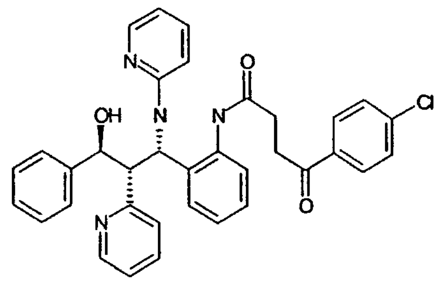 | C35 H31 Cl N4 O3 | 591,1 | 592.1 M+H+ |
| 37 | 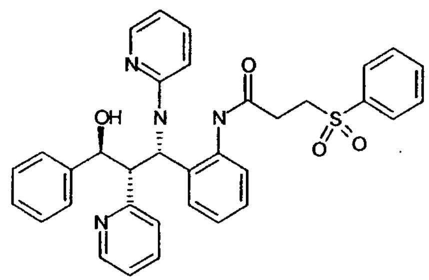 | C34 H32 N4 O4 S | 592,7 | 593.7 M+H+ |
| 38 | 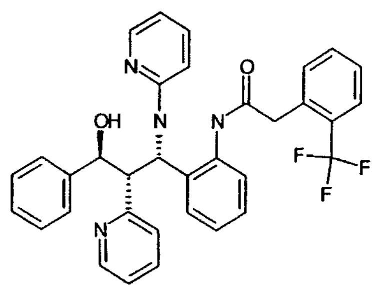 | C34 H29 F3 N4 O2 | 582,6 | 583.6 M+H+ |

| | | | | |
|---|---|---|---|---|
| 39 | 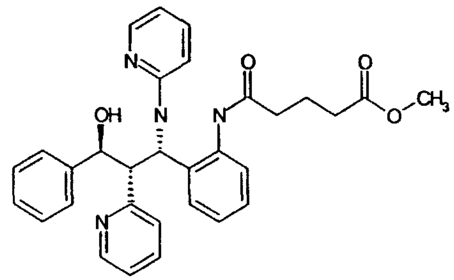 | C31 H32 N4 O4 | 524,6 | 525.6 M+H+ |
| 40 | 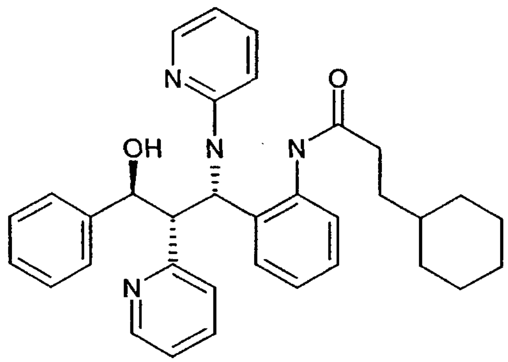 | C34 H38 N4 O2 | 534,7 | 535.6 M+H+ |
| 41 | 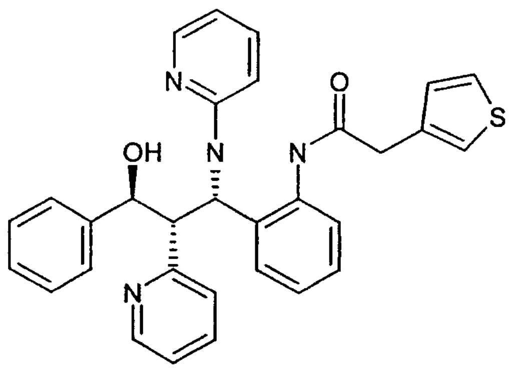 | C31 H28 N4 O2 S | 520,7 | 521.7 M+H+ |

| | | | | |
|---|---|---|---|---|
| 42 | 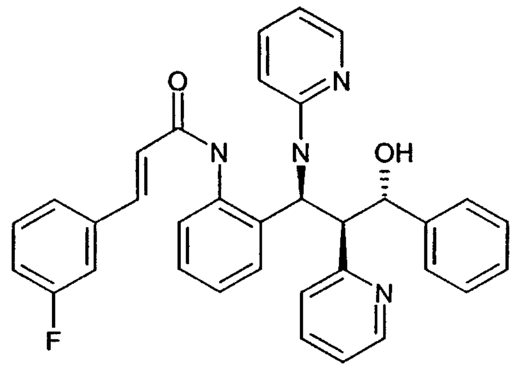 | $C_{34}H_{29}FN_4O_2$ | 544,6 | 545.6 $M+H^+$ |
| 43 | 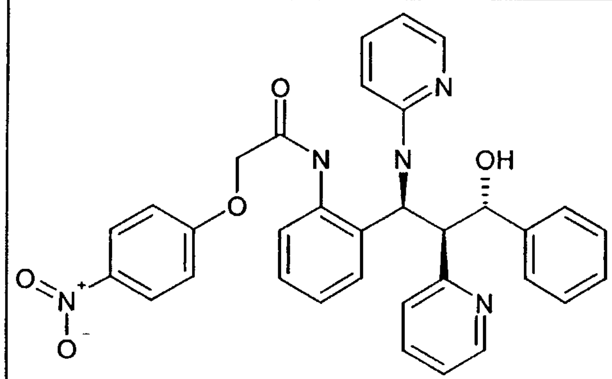 | $C_{33}H_{29}N_5O_5$ | 575,6 | 576.6 $M+H^+$ |

| | | | | |
|---|---|---|---|---|
| 44 | 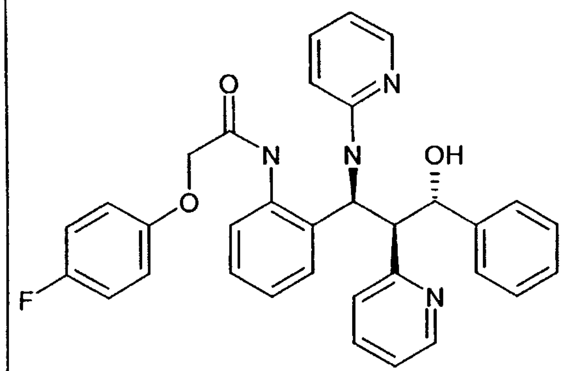 | C33 H29 F N4 O3 | 548,6 | 549.6 M+H⁺ |
| 45 | 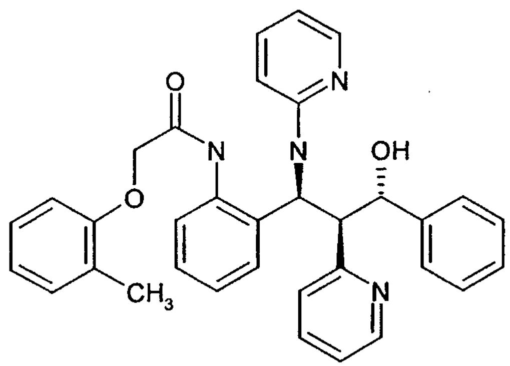 | C34 H32 N4 O3 | 544,7 | 545.6 M+H⁺ |

| | | | | |
|---|---|---|---|---|
| 46 | 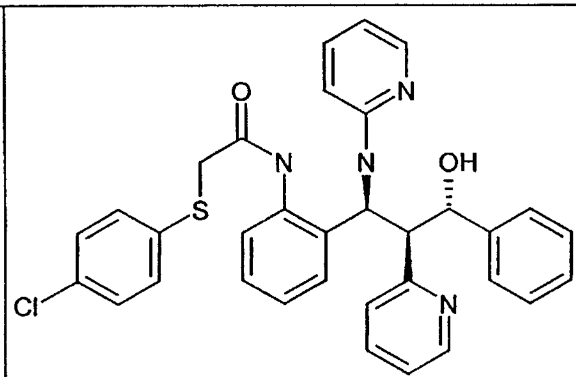 | C33 H29 Cl N4 O2 S | 581,1 | 582.1 M+H+ |
| 47 | 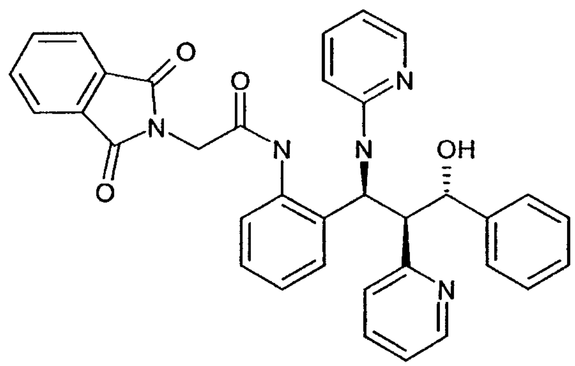 | C35 H29 N5 O4 | 583,6 | 584.6 M+H+ |

| | | | | |
|---|---|---|---|---|
| 48 | 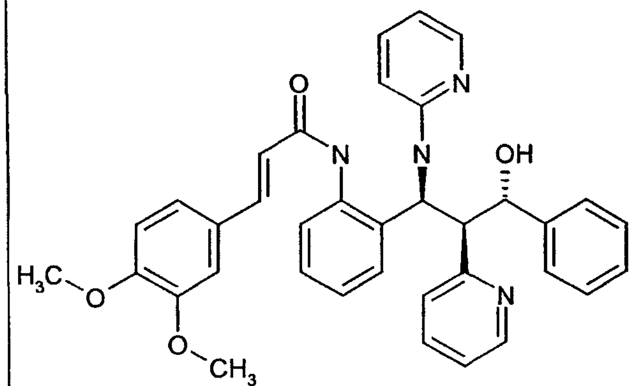 | C36 H34 N4 O4 | 586,7 | 587.7 M+H$^+$ |
| 49 | 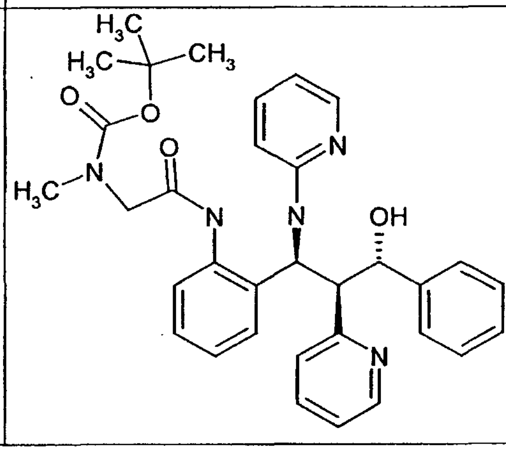 | C33 H37 N5 O4 | 567,7 | 568.7 M+H$^+$ |

| | | | | |
|---|---|---|---|---|
| 50 | 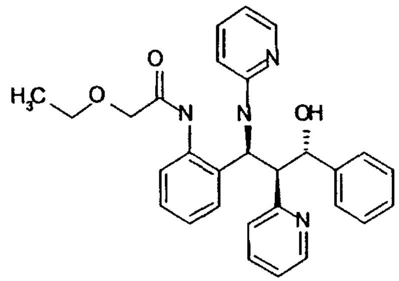 | C29 H30 N4 O3 | 482,6 | 483.6 M+H$^+$ |
| 51 | 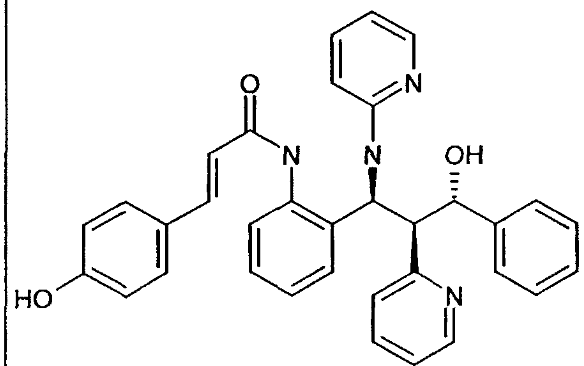 | C34 H30 N4 O3 | 542,6 | 543.6 M+H$^+$ |
| 52 | 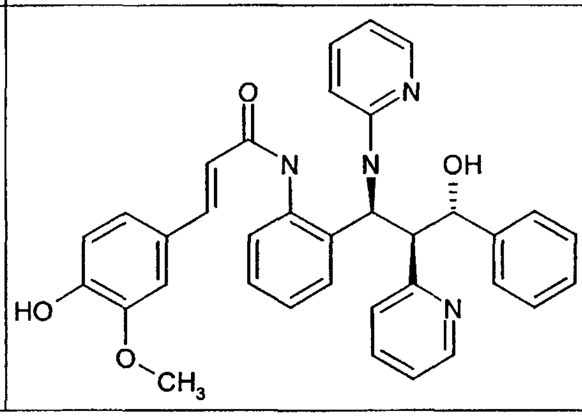 | C35 H32 N4 O4 | 572,7 | 573.7 M+H$^+$ |

| | | | | |
|---|---|---|---|---|
| 53 | 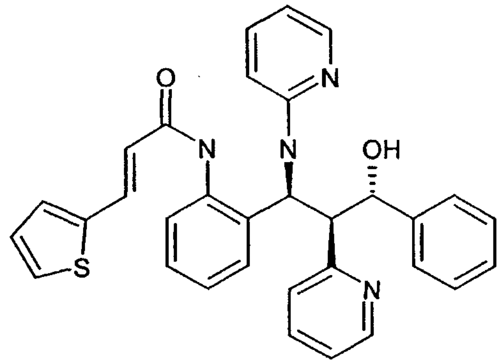 | C32 H28 N4 O2 S | 532,7 | 533.7 M+H$^+$ |
| 54 | 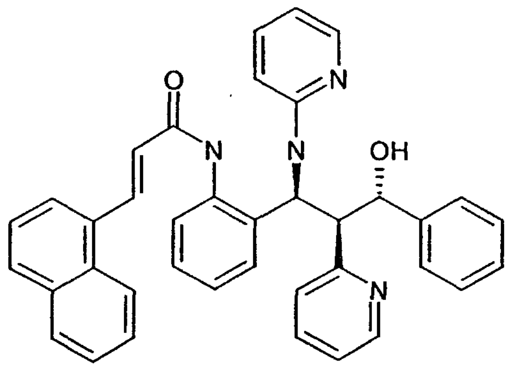 | C38 H32 N4 O2 | 576,7 | 577.7 M+H$^+$ |
| 55 | 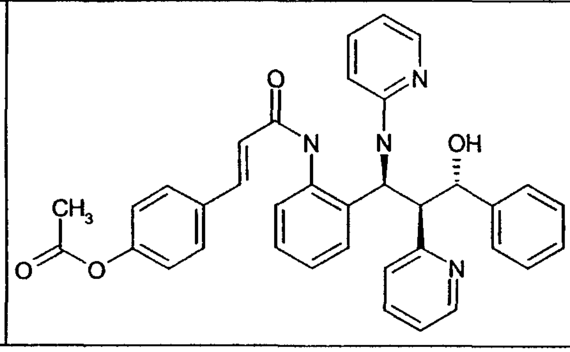 | C36 H32 N4 O4 | 584,7 | 585.7 M+H$^+$ |

| | | | | |
|---|---|---|---|---|
| 56 | 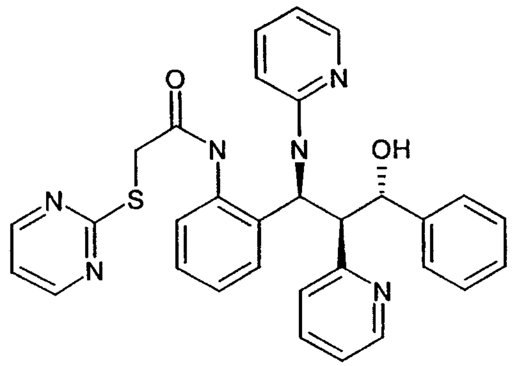 | C31 H28 N6 O2 S | 548,7 | 549.7 M+H$^+$ |
| 57 | 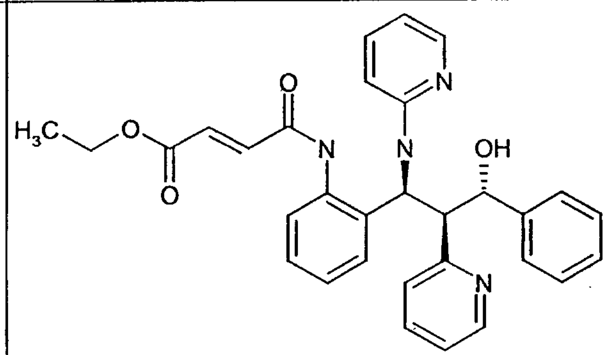 | C31 H30 N4 O4 | 522,6 | 523.6 M+H$^+$ |

| | | | | |
|---|---|---|---|---|
| 58 | 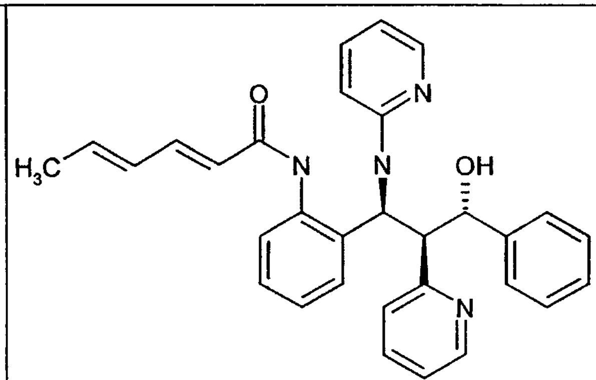 | C31 H30 N4 O2 | 490,6 | 491.6 M+H$^+$ |
| 59 | 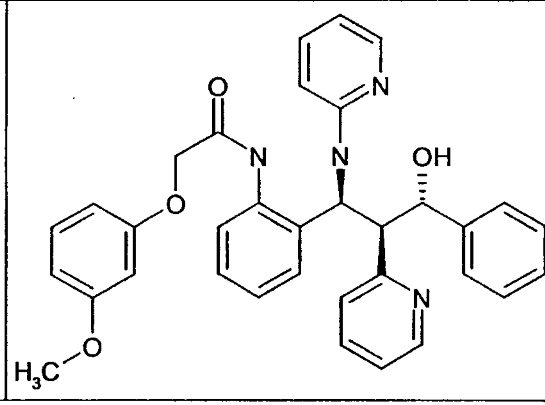 | C34 H32 N4 O4 | 560,7 | 561.7 M+H$^+$ |

| | | | | |
|---|---|---|---|---|
| 60 | 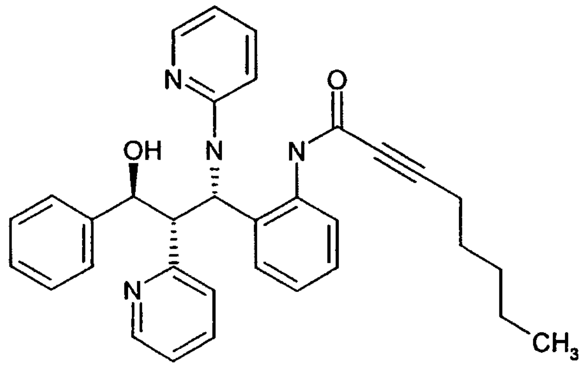 | C33 H34 N4 O2 | 518,7 | 519.7 $M+H^+$ |
| 61 | 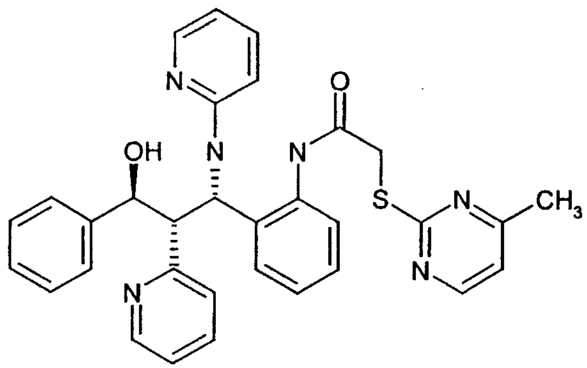 | C32 H30 N6 O2 S | 562,7 | 563.7 $M+H^+$ |

| | | | | |
|---|---|---|---|---|
| 62 | 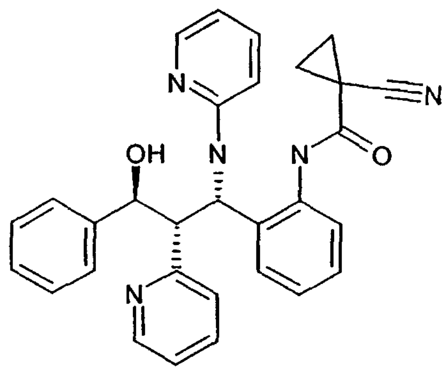 | C30 H27 N5 O2 | 489,6 | 490.6 M+H$^+$ |
| 63 | 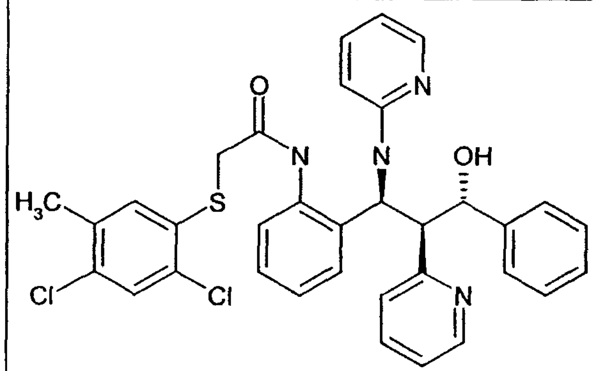 | C34 H30 Cl2 N4 O2 S | 629,6 | 530.6 M+H$^+$ |

| | | | | |
|---|---|---|---|---|
| 64 | 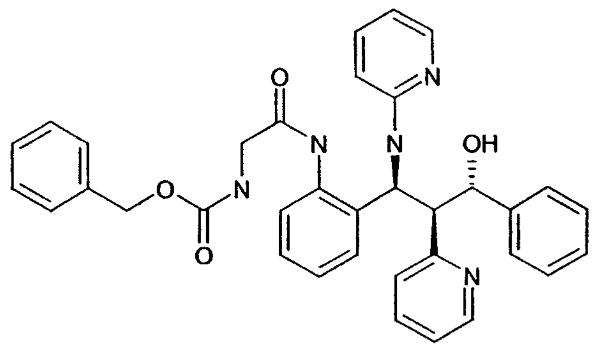 | C35 H33 N5 O4 | 587,7 | 588.7 M+H+ |
| 65 | 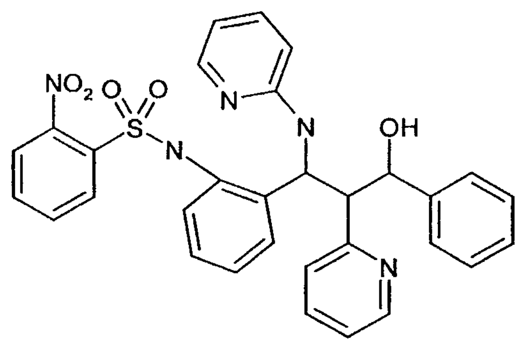 | C31 H27 N5 O5 S | 581,7 | 582.6 M+H+ |

| | | | | |
|---|---|---|---|---|
| 66 | 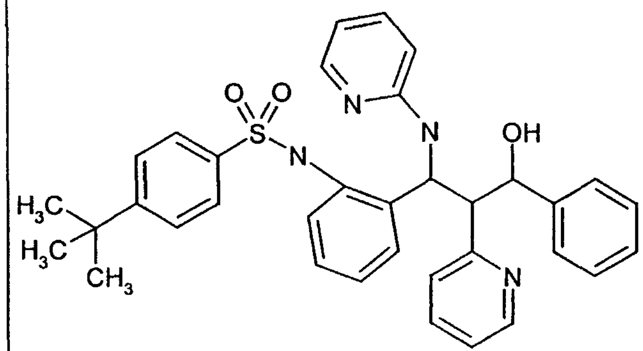 | C35 H36 N4 O3 S | 592,8 | 593.8 M+H+ |
| 67 | 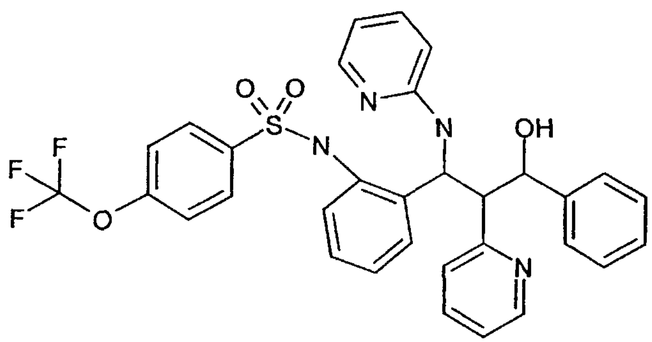 | C32 H27 F3 N4 O4 S | 620,6 | 521.6 M+H+ |
| 68 | 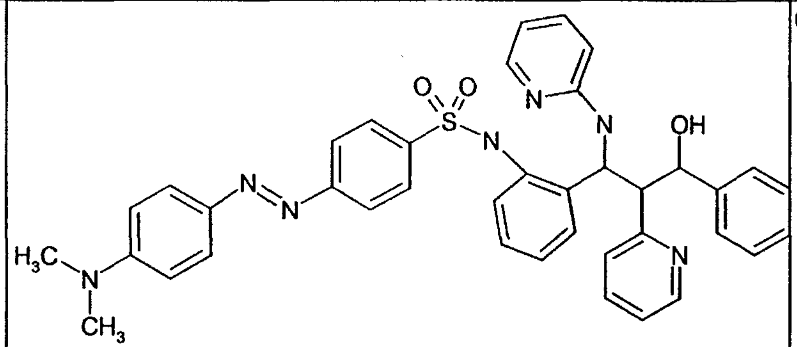 | C39 H37 N7 O3 S | 683,8 | 584.8 M+H+ |

| | | | | |
|---|---|---|---|---|
| 69 | 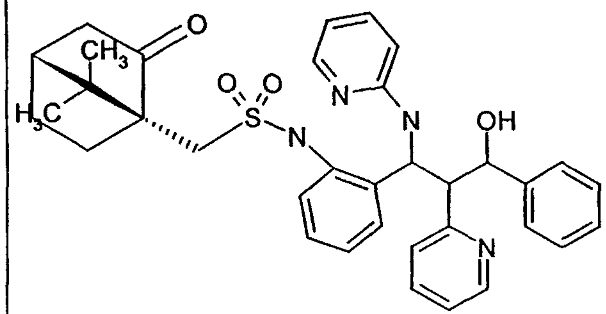 | C35 H38 N4 O4 S | 610,8 | 611.6 M+H+ |
| 70 | 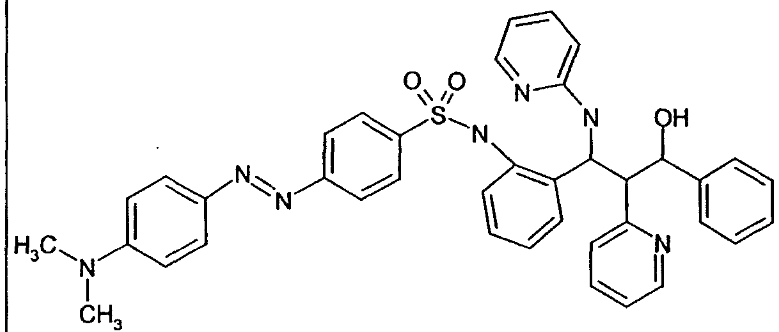 | C34 H29 N5 O3 S | 587,7 | 588.7 M+H+ |
| 71 | 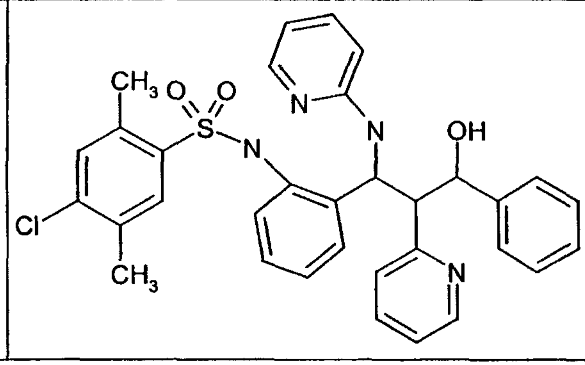 | C33 H31 Cl N4 O3 S | 599,2 | 600.2 M+H+ |

| | | | | |
|---|---|---|---|---|
| 72 | 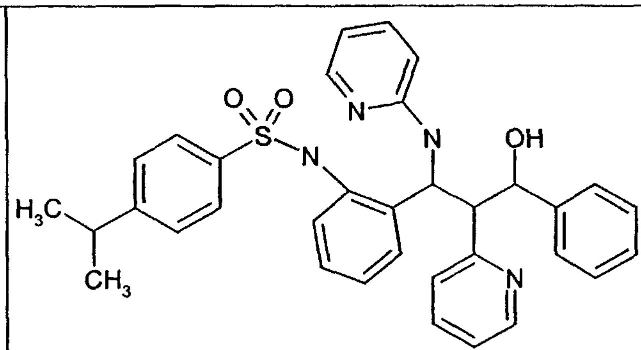 | C34 H34 N4 O3 S | 578,7 | 579.7 $M+H^+$ |
| 73 | 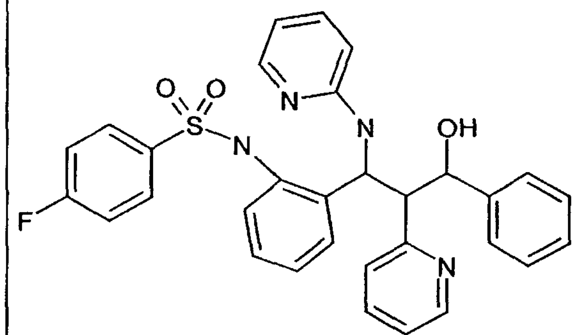 | C31 H27 F N4 O3 S | 554,6 | 555.6 $M+H^+$ |
| 74 | 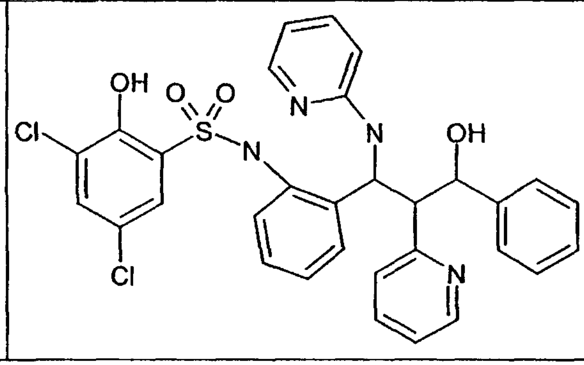 | C31 H26 Cl2 N4 O4 S | 621,5 | 522.5 $M+H^+$ |

| | | | | |
|---|---|---|---|---|
| 75 | 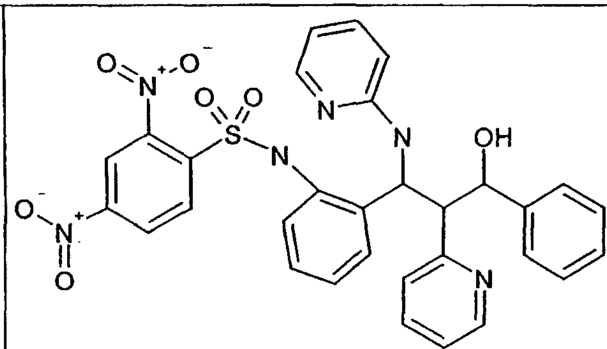 | C31 H26 N6 O7 S | 626,6 | 627.6 M+H$^+$ |
| 76 | 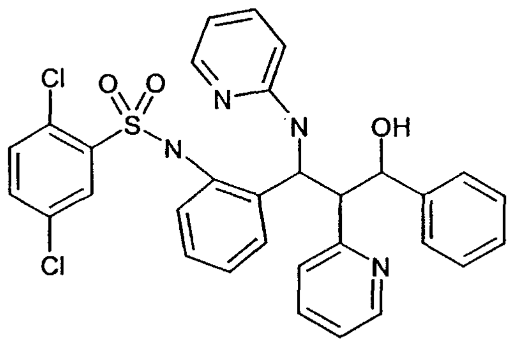 | C31 H26 Cl2 N4 O3 S | 605,5 | 606.5 M+H$^+$ |
| 77 | 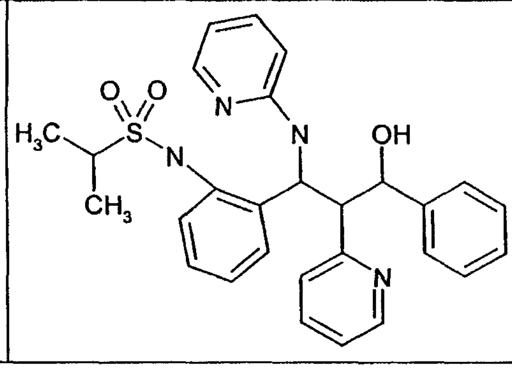 | C28 H30 N4 O3 S | 502,6 | 503.6 M+H$^+$ |

| | | | | |
|---|---|---|---|---|
| 78 | 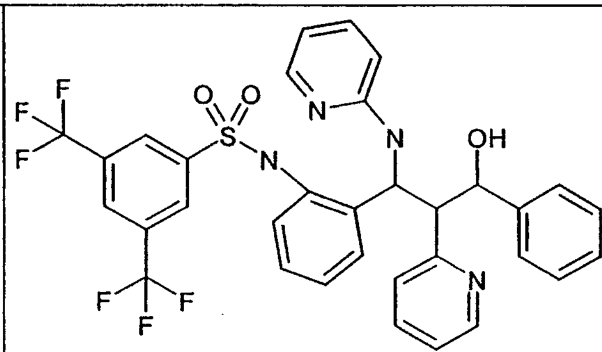 | C33 H26 F6 N4 O3 S | 672,6 | 573.6 M+H+ |
| 79 | 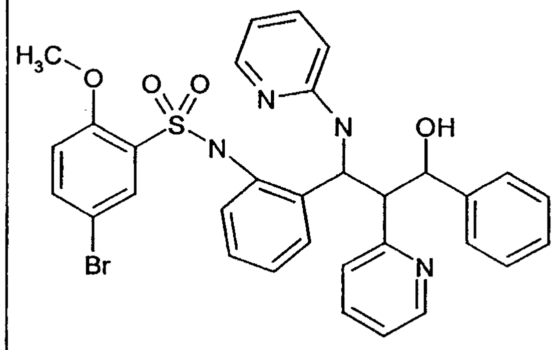 | C32 H29 Br N4 O4 S | 645,6 | 646.6 M+H+ |
| 80 | 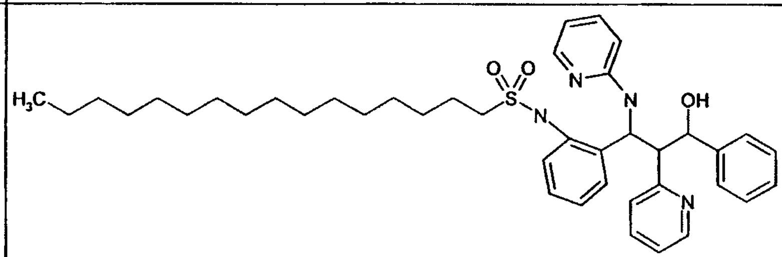 | C41 H56 N4 O3 S | 685,0 | 686.0 M+H+ |

| | | | | |
|---|---|---|---|---|
| 81 | 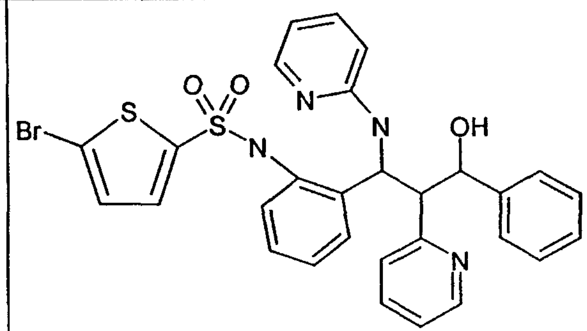 | C29 H25 Br N4 O3 S2 | 621,6 | 622.6 M+H+ |
| 82 | 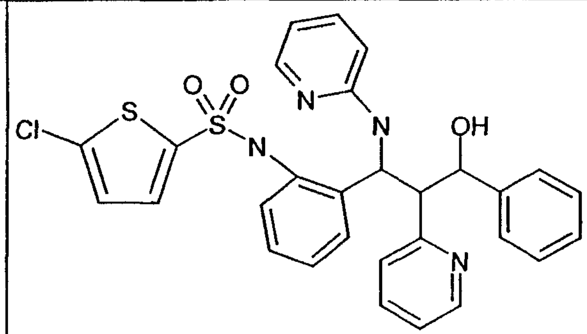 | C29 H25 Cl N4 O3 S2 | 577,1 | 578.1 M+H+ |
| 83 | 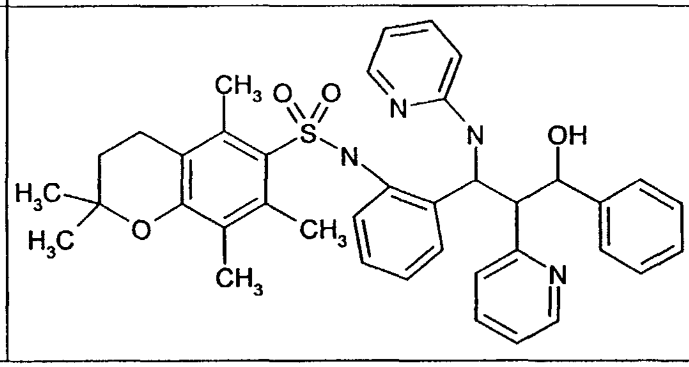 | C39 H42 N4 O4 S | 662,9 | 663.9 M+H+ |

| | | | | |
|---|---|---|---|---|
| 84 | 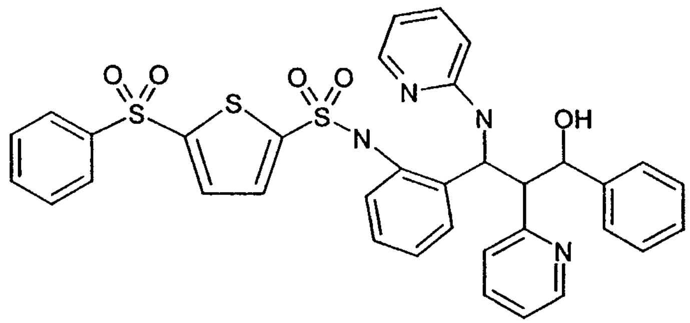 | C35 H30 N4 O5 S3 | 682,8 | 683.8 M+H+ |
| 85 | 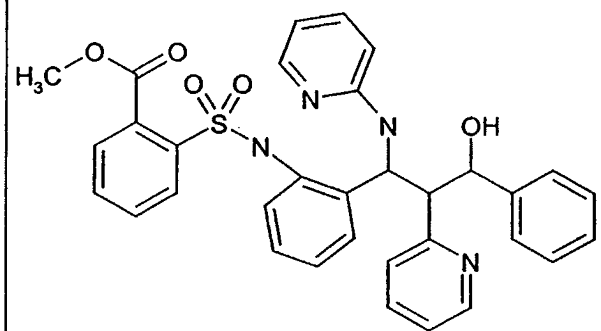 | C33 H30 N4 O5 S | 594,7 | 595.7 M+H+ |
| 86 | 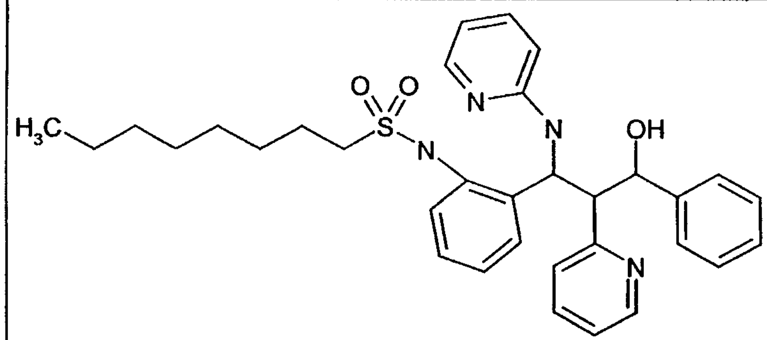 | C33 H40 N4 O3 S | 572,8 | 573.8 M+H+ |

| | | | | |
|---|---|---|---|---|
| 87 | 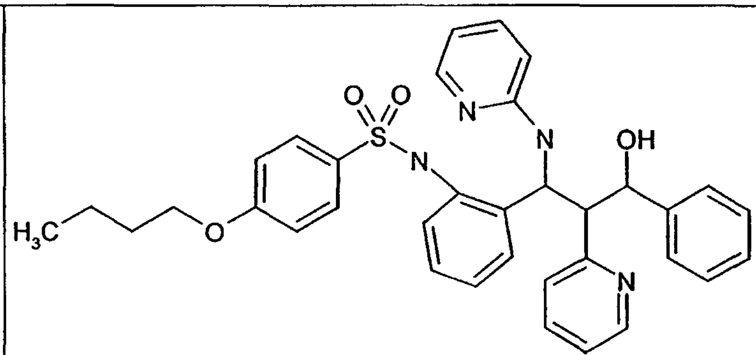 | C35 H36 N4 O4 S | 608,8 | 609.8 M+H⁺ |
| 88 | 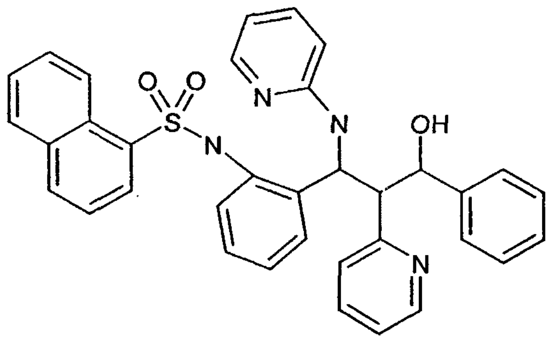 | C35 H30 N4 O3 S | 586,7 | 587.7 M+H⁺ |
| 89 | 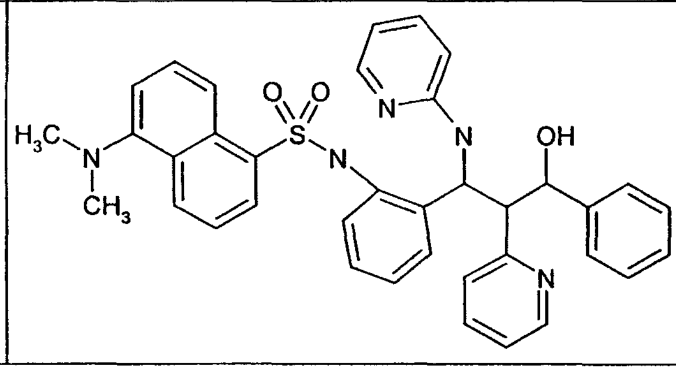 | C37 H35 N5 O3 S | 629,8 | 630.8 M+H⁺ |

| | | | | |
|---|---|---|---|---|
| 90 | 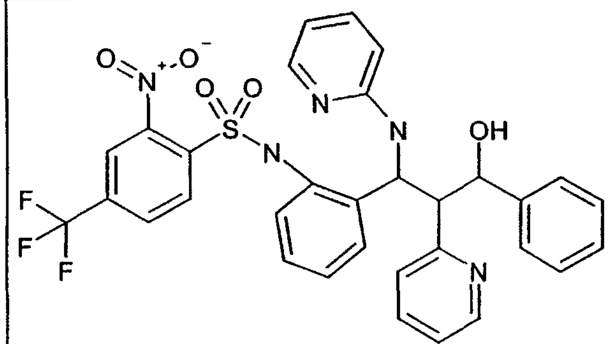 | C32 H26 F3 N5 O5 S | 649,6 | 650.6 M+H$^+$ |
| 91 | 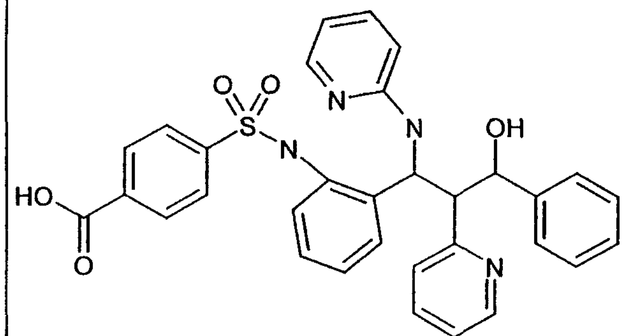 | C32 H28 N4 O5 S | 580,7 | 581.7 M+H$^+$ |
| 92 | 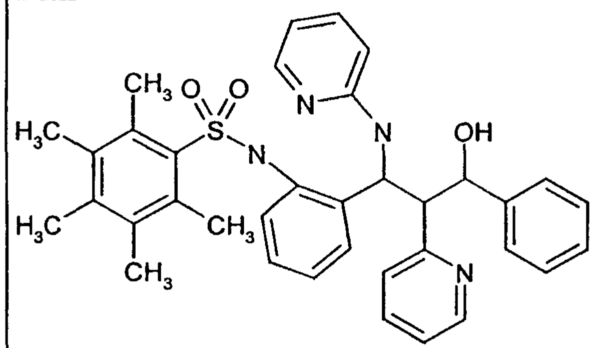 | C36 H38 N4 O3 S | 606,8 | 607.8 M+H$^+$ |

| | | | | |
|---|---|---|---|---|
| 93 | 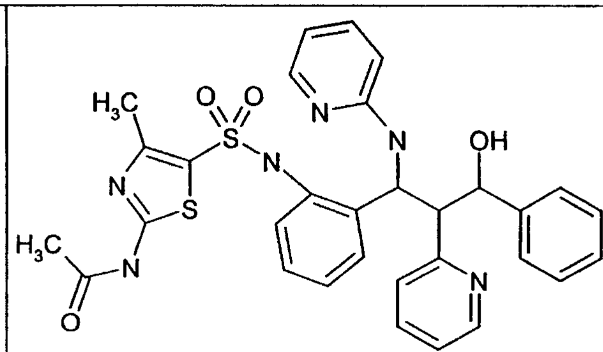 | C31 H30 N6 O4 S2 | 614,7 | 615.7 M+H+ |
| 94 | 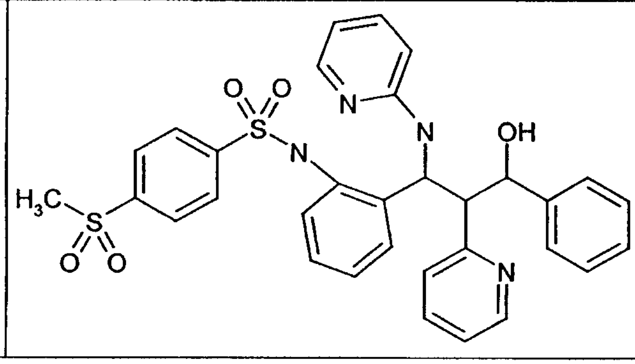 | C32 H30 N4 O5 S2 | 614,7 | 615.7 M+H+ |

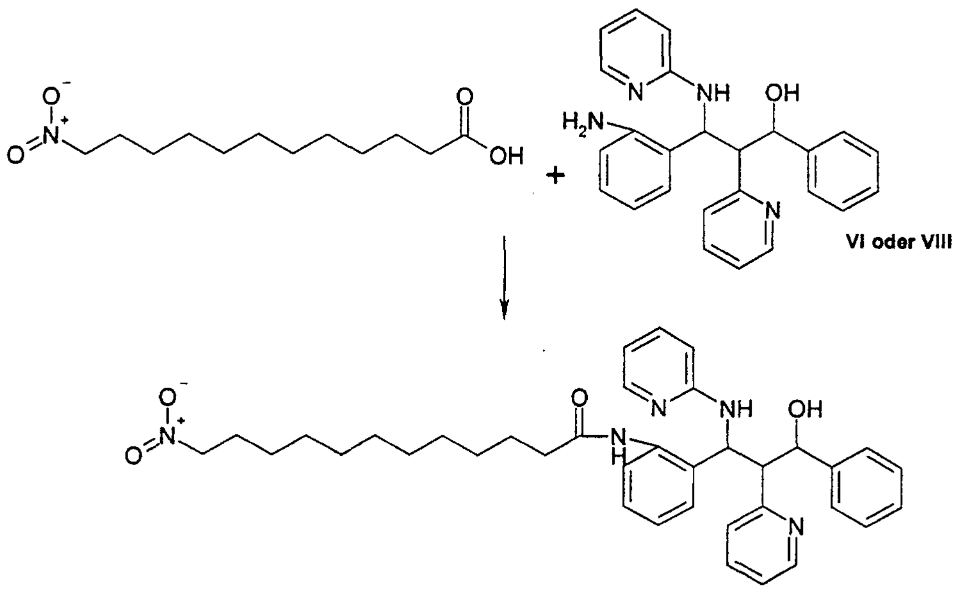


Beispiel 95

1.6 g des Amins der Formel VI oder VIII und 0.98 g 12-Nitrododecansäure wurden in 30 ml Dimethylformamid gelöst . Dazu gab man 1.6 g O-((ETHOXYCARBONYL)CYANOMETHYLENEAMINO)-N,N,N',N'-TETRAMET HYLURONIUM TETRAFLUOROBORATE (TOTU), 0.6 g Ethyl-(hydroxyimino)-cyanacetat und 1.6 ml N-Ethylmorpholin und rührte ca. 2 h bei Raumtemp. Nach beendeter Umsetzung (DC) wurde das Reaktionsgemisch mit 500 ml Wasser und 200 ml Dichlormethan ausgerührt, die org. Phase abgetrennt, getrocknet und i. Vak. eingeengt. Nach Säulenchromatographie (SC; $SiO_2$, Ethylacetat/n-Heptan = 2:1) erhielt man das Amid als zähes Öl. Summenformel: C37H45N5O4; (623.4) ; MS(FAB): 624.4 $M+H^+$

Beispiel 96

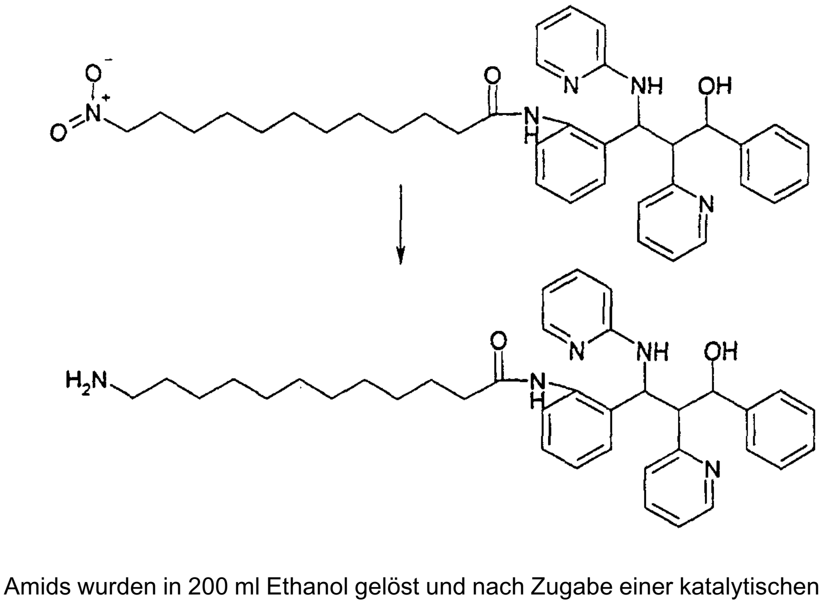

2.2 g des Amids wurden in 200 ml Ethanol gelöst und nach Zugabe einer katalytischen Menge Raney-Nickel (wäßr. Suspension) in einer Schüttelente bei Normaldruck und Raumtemp. hydriert. Man saugt über eine Klärschicht ab, engte ein und erhielt nach SC ($SiO_2$, Dichlormethan/Methanol/Ammoniak= 90:10:1) Beispiel 95. Summenformel C37H47N5O2 (593.8) MS(FAB): 595 $M+H^+$

Beispiel 97

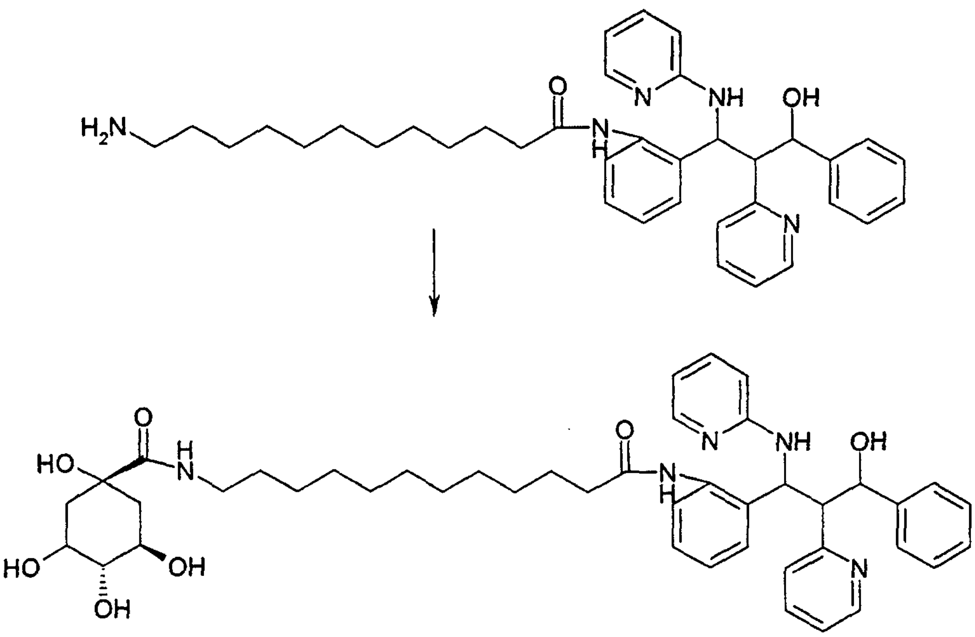

1,2 g Beispiel 96, 390 mg Chinasäure und 330 mg N-Hydroxy-benzotriazol wurden in 100 ml Tetrahydrofuran gelöst und mit 500 mg Dicyclohexylcarbodiimid versetzt. Man rührte 17 h bei Raumtemp., filtriert ab und engt ein. Der Rückstand wurde mit ca. 500 ml Ethylacetat aufgenommen und nacheinander mit $NaHCO_3$-Lsg., 2N Citronensäure, $NaHCO_3$-Lsg. und Wasser ausgeschüttelt, getrocknet und eingeengt. Nach Säulenfiltration (Ethylacetat/Methanol=9: 1) erhielt man Beispiel 3, Schmp. 95°C. Summenformel: C44H47N5O7 (768), MS(FAB): 768.4 $M+H^+$

Beispiel 98

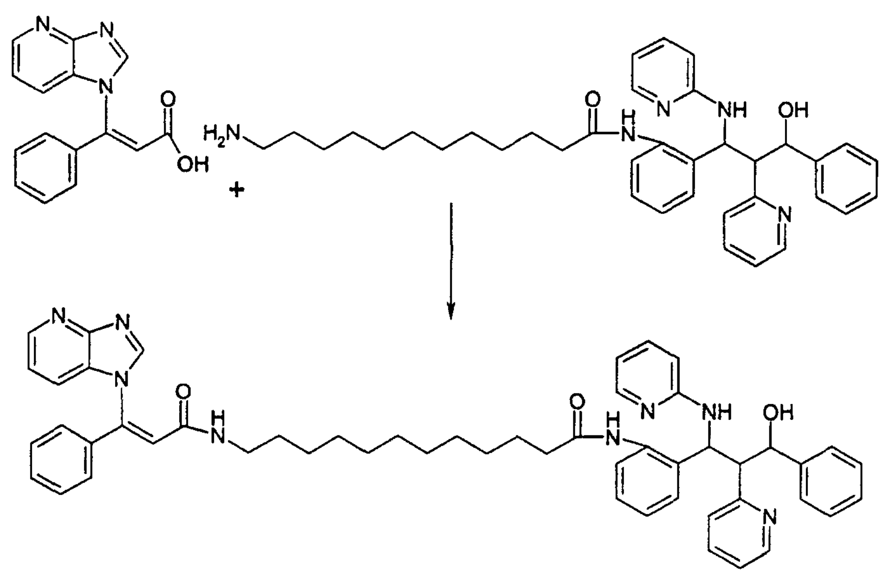

593 mg Beispiel 96 und 265 mg der o.g. Carbonsäure wurden in 20 ml DMF gelöst. Dazu gibt man: 600 mg

TOTU, 300 mg Ethyl-hydroxy-imino-cyanacetat und 1 ml N-Ethylmorpholin und rührte ca. 2 h bei Raumtemp. Nach beendeter Reaktion (DC) versetzte man mit Ethylacetat und wusch je 2mal mit Wasser und $NaHCO_3$-Lsg., engt die organische Phase ein und reinigt den Rückstand durch SC (SiO2, Ethylacetat/Methanol = 9:1). Man erhält das Amid Beispiel 4 vom Schmp. 105°C. Summenformel: C52H56N8O3 (840.5); MS: 842 ($M+H^+$).

Analog zu Beispiel 4 werden folgende Substanzen aus dem Amin Beispiel 2 und der entsprechenden Carbonsäure hergestellt:

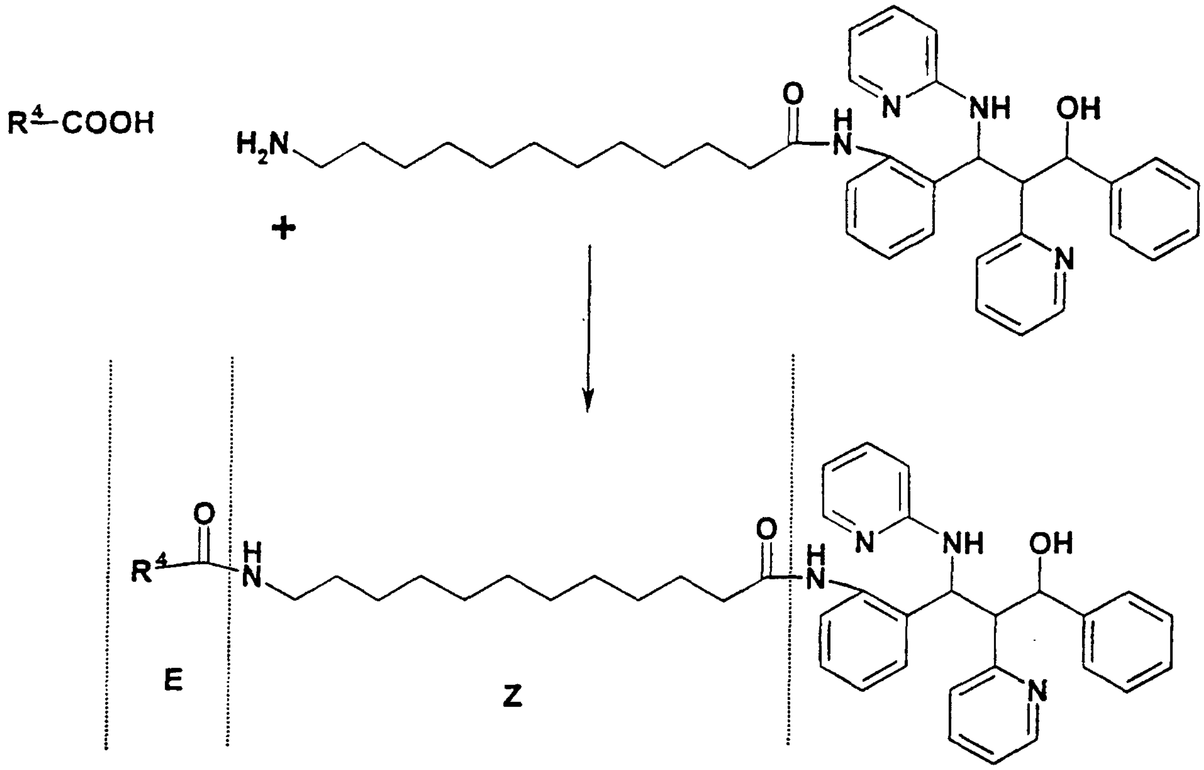

Tabelle 5

| Beispiel | $R^4$-COOH | Summenformel (Massenzahl) | MS (FAB) |
|---|---|---|---|
| 99 | 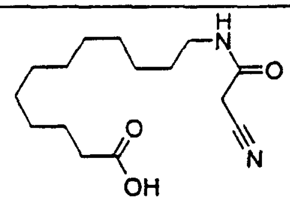 | C40 H48 N6 O3<br>660.4 | 661.5 ($M+H^+$) |
| 100 | 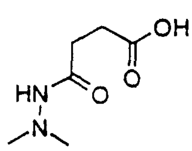 | C43 H57 N7 O4<br>735.5 | 737 ($M+H^+$) |

| | | | |
|---|---|---|---|
| 101 | 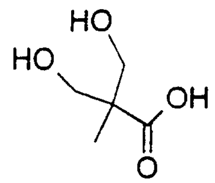 | C42 H55 N5 O5<br>709.4 | 711 (M+H$^+$) |
| 102 | 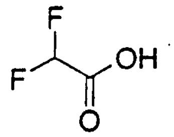 | C39 H47 F2 N5 O3<br>671.4 | 673 (M+H$^+$) |
| 103 | 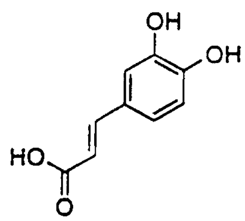 | C46 H53 N5 O5<br>755.4 | 757(M+H$^+$) |
| 104 | 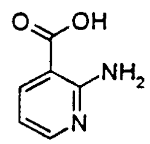 | C43 H51 N7 O3<br>713.4 | 715 (M+H$^+$) |
| 105 | 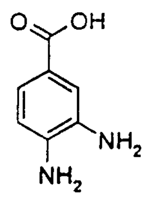 | C44 H53 N7 O3<br>727.4 | 729 (M+H$^+$) |
| 106 | 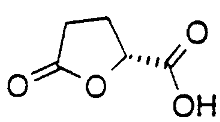 | C42 H51 N5 O5<br>705.4 | 707 (M+H$^+$) |
| 107 | 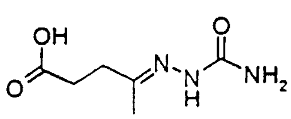 | C43 H56 N8 O4<br>748.4 | 750 (M+H$^+$) |

| | | | |
|---|---|---|---|
| 108 | 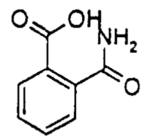 | C45 H52 N6 O4<br>740.4 | 741 (M+H$^+$) |
| 109 | 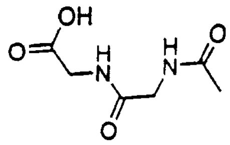 | C43 H55 N7 O5<br>749.4 | 751 (M+H$^+$) |
| 110 | 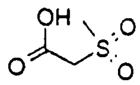 | C40 H51 N5 O5 S<br>713.4 | 715 (M+H$^+$) |
| 111 | 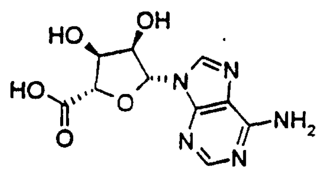 | C47 H56 N10 O6<br>856.4 | 858 (M+H$^+$) |
| 112 | 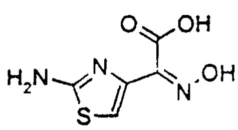 | C42 H50 N8 O4 S<br>762.4 | 764 (M+H$^+$) |
| 113 | 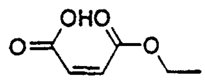 | C43 H53 N5 O5<br>719.4 | 721 (M+H$^+$) |
| 114 | 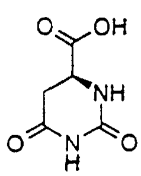 | C42 H51 N7 O5<br>733.4 | 735 (M+H$^+$) |

| | | | |
|---|---|---|---|
| 115 | 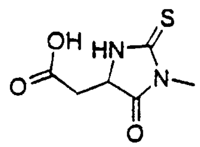 | C43 H53 N7 O4 S<br>763.4 | 765 (M+H⁺) |
| 116 | 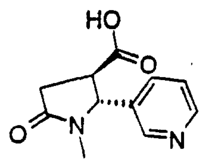 | C48 H57 N7 O4<br>795.5 | 797 (M+H⁺) |
| 117 | 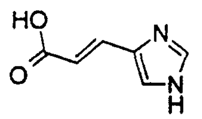 | C43 H51 N7 O3<br>713.4 | 715 (M+H⁺) |
| 118 | 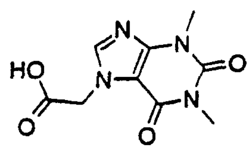 | C46 H55 N9 O5<br>813.4 | 815 (M+H⁺) |
| 119 | 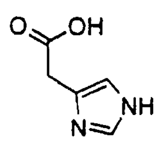 | C42 H51 N7 O3<br>701.4 | 703 (M+H⁺) |
| 120 | 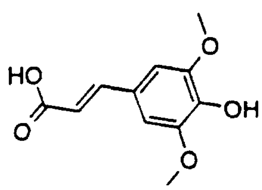 | C48 H57 N5 O6<br>799.4 | 801 (M+H⁺) |
| 121 | 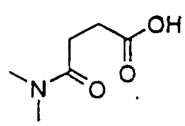 | C43 H56 N6 O4<br>720.4 | 722 (M+H⁺) |

| | | | |
|---|---|---|---|
| 122 | 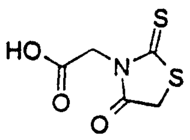 | C42 H50 N6 O4 S2<br>766.3 | 768 (M+H⁺) |
| 123 | 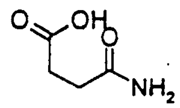 | C41 H52 N6 O4<br>692.4 | 694 (M+H⁺) |
| 124 | 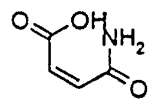 | C41 H50 N6 O4<br>690.4 | 692 (M+H⁺) |
| 125 | 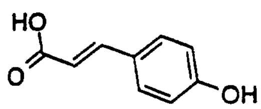 | C46 H53 N5 O4<br>739.4 | 741 (M+H⁺) |

Tabelle 6:

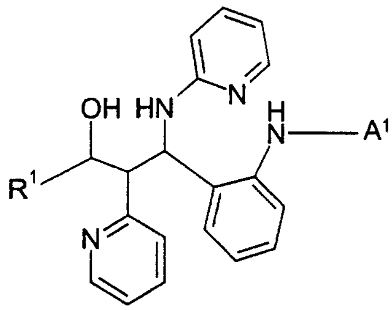

| Beispiel | $R^1$ | Aminosäurerest $A^1$ | Summenformel (Molmasse) | MS |
|---|---|---|---|---|
| 133 I | 3,5-Dimethyl-isoxazol-4-yl | D-Lys(Boc) | $C_{35}H_{45}N_7O_5$ (643,8) | 644,4 (FAB), M + $H^+$ |
| 134 I | 2,4-Dimethyl-thiazol-5-yl | D-Lys(Boc) | $C_{35}H_{45}N_7O_4S$ (659,9) | 660,4 (ESI), M + $H^+$ |
| 135 | 2,5-Dimethyl-oxazol-4-yl | D-Lys(Boc) | $C_{35}H_{45}N_7O_5$ (643,8) | 644,4 (FAB), M + $H^+$ |

Tabelle 7:

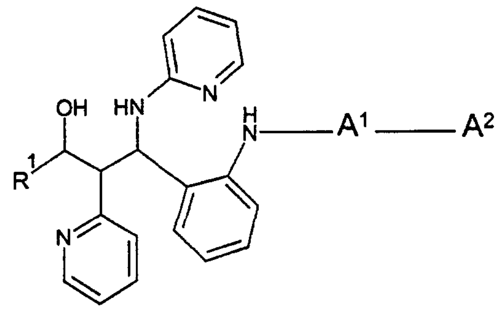

| Beispiel | $R^1$ | Aminosäurerest $A^1$ | Aminosäurerest $A^2$ | Summenformel (Molmasse) | MS |
|---|---|---|---|---|---|
| 143 (stark unpolar) | 3,5-Dimethyl-isoxazolyl-4-yl | L-Prolin | L-Phenylalanin | $C_{38}H_{41}N_7O_4$ (659,8) | 660,3 (ESI), M + $H^+$ |
| 144 (stark unpolar) | 3,5-Dimethyl-isoxazolyl-4-yl | D-Lys(Boc) | D-Lys(Boc) | $C_{46}H_{65}N_9O_8$ (872,1) | 772,4 (FAB), M + $H^+$ |
| 145 (stark unpolar) | 2,5-Dimethyl-oxazol-4-yl | D-Lys(Boc) | D-Lys(Boc) | $C_{46}H_{65}N_9O_8$ (872,1) | 772,5 (FAB), M + $H^+$ |
| 146 (stark unpolar) | 5-Methyl-isoxazol-3-yl | D-Lys(Boc) | D-Lys(Boc) | $C_{45}H_{63}N_9O_8$ (858,1) | 858,5 (FAB), M + $H^+$ |
| 147 (stark unpolar) | 2,4-Dimethyl-thiazol-5-yl | D-Lys(Boc) | D-Lys(Boc) | $C_{46}H_{65}N_9O_7S$ (888,2) | 888,6 (ESI) M + $H^+$ |
| 148 unpolar | 2,4-Dimethyl-thiazol-5-yl | D-Lys(Boc) | D-Lys(Boc) | $C_{46}H_{65}N_9O_7S$ (888,2) | 888,4 (FAB) M + $H^+$ |
| 149 (mittel-polar) | 2,4-Dimethyl-thiazol-5-yl | D-Lys(Boc) | D-Lys(Boc) | $C_{46}H_{65}N_9O_7S$ (888,2) | 888,6 (ESI) M + $H^+$ |
| 150 S983499 (polar) | 2,4-Dimethyl-thiazol-5-yl | D-Lys(Boc) | D-Lys(Boc) | $C_{46}H_{65}N_9O_7S$ (888,2) | 888,4 (FAB) M + $H^+$ |
| 151 (stark unpolar) | 2,4-Dimethyl-thiazol-5-yl | D-Lys(Boc) | L-Phenylalanin | $C_{44}H_{54}N_8O_5S$ (807,0) | 807,5 (ESI) M + $H^+$ |
| 32 (stark unpolar) | 2,4-Dimethyl-thiazol-5-yl | L-Prolin | L-Phenylalanin | $C_{38}H_{41}N_7O_3S$ (675,9) | 676,4 (FAB) M + $H^+$ |

## Patentansprüche

**1.** Verbindungen der Formel I,

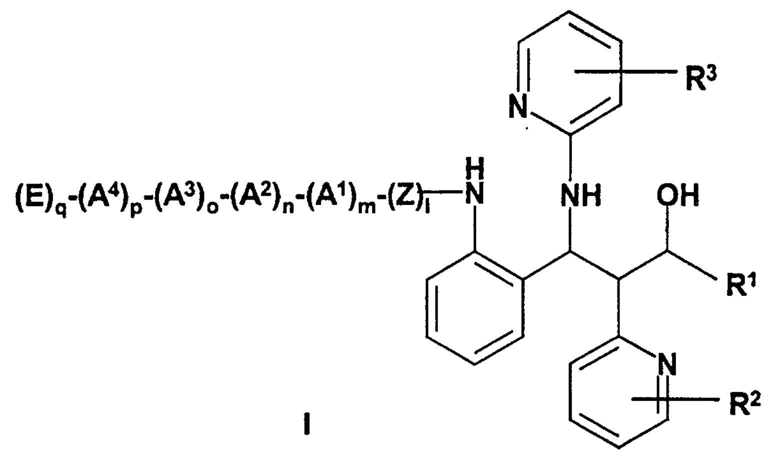

I

| | |
|---|---|
| Z | -NH-($C_1$-$C_{16}$-Alkyl)-(C=O)-;<br>-(C=O)-($C_1$-$C_{16}$-Alkyl)-(C=O)- ;<br>- (C=O)-Phenyl-(C=O)- ; |
| $A^1$, $A^2$, $A^3$,$A^4$, | unabhängig voneinander einen Aminosäurerest, einen Aminosäurerest der ein- oder mehrfach substituiert ist durch Aminosäure-Schutzgruppen; |
| E | -$SO_2$-$R^4$, -CO-$R^4$; |
| $R^1$ | Phenyl, Thiazolyl, Oxazolyl, Thienyl, Thiophenyl, Furanyl, Pyridyl, Pirimidyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]$_2$, CONH($C_3$-$C_6$)Cycloalkyl, $NH_2$, NH-CO-($C_1$-$C_6$)-Alkyl, NH-CO-Phenyl; |
| $R^2$ | H, OH, $CH_2OH$, OMe, |
| $R^3$ | H, F, Methyl, OMe; |
| $R^4$ | -($C_5$-$C_{16}$-Alkyl), -($C_0$-$C_{16}$-Alkylen)-$R^5$, -(C=O)-($C_0$-$C_{16}$-Alkylen)-$R^5$, -(C=O)-($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-S(O)$_{0-2}$-$R^5$, -($C_1$-$C_4$-Alkylen)-O-$R^5$, -($C_1$-$C_4$-Alkylen)-NH-$R^5$; |
| $R^5$ | -COO-$R^6$, -(C=O)- $R^6$, -($C_1$-$C_6$-Alkylen)-$R^7$, -($C_1$-$C_6$-Alkenylen)-$R^7$ , (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, COO($C_1$-$C_6$)Alkyl, COO($C_3$-$C_6$)Cycloalkyl, $CONH_2$, CONH($C_1$-$C_6$)Alkyl, CON[($C_1$-$C_6$)Alkyl]$_2$, CONH($C_3$-$C_6$)Cycloalkyl, $NH_2$, NH-CO-($C_1$-$C_6$)-Alkyl, NH-CO-Phenyl, Pyridyl; |
| $R^6$ | H, -($C_1$-$C_6$)Alkyl; |
| $R^7$ | H, (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, |

$COO(C_1-C_6)Alkyl$, $COO(C_3-C_6)Cycloalkyl$, $CONH_2$, $CONH(C_1-C_6)Alkyl$, $CON[(C_1-C_6)Alkyl]_2$, $CONH(C_3-C_6)Cycloalkyl$, $NH_2$, NH-CO-$(C_1-C_6)$-Alkyl, NH-CO-Phenyl;

l, q, m, n, o, p — unabhängig voneinander 0 oder 1, mit l+q+m+n+o+p größer oder gleich 1;

sowie deren pharmazeutisch verträgliche Salze.

**2.** Verbindungen der Formel I, gemäß Anspruch 1, **dadurch gekennzeichnet, daß** darin bedeuten

Z — -NH-($C_1$-$C_{16}$-Alkyl)-(C=O)-,
-(C=O)-($C_1$-$C_{16}$-Alkyl)-(C=O)- ,
-(C=O)-Phenyl-(C=O)- ;

$A^1$, $A^2$, $A^3$,$A^4$, — unabhängig voneinander einen Aminosäurerest, einen Aminosäurerest der ein- oder mehrfach substituiert ist durch Aminosäure-Schutzgruppen;

E — $-SO_2-R^4$, $-CO-R^4$;

$R^1$ — Phenyl, Thiazolyl, Oxazolyl, Thienyl, Thiophenyl, Furanyl, Pyridyl, Pirimidyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)Cycloalkyl, COOH, $COO(C_1-C_6)Alkyl$, $COO(C_3-C_6)Cycloalkyl$, $CONH_2$, $CONH(C_1-C_6)Alkyl$, $CON[(C_1-C_6)Alkyl]2$, $CONH(C_3-C_6)Cycloalkyl$, $NH_2$, NH-CO-($C_1$-$C_6$)-Alkyl, NH-CO-Phenyl,;

$R^2$ — H, OH, $CH_2OH$, OMe;

$R^3$ — H, F, Methyl, OMe;

$R^4$ — -($C_5$-$C_{16}$-Alkyl), -($C_0$-$C_{16}$-Alkylen)-$R^5$, -(C=O)-($C_0$-$C_{16}$-Alkylen)-$R^5$, -(C=O)-($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-$S(0)_{0-2}$-$R^5$, -($C_1$-$C_4$-Alkylen)-O-$R^5$, -($C_1$-$C_4$-Alkylen)-NH-$R^5$;

$R^5$ — -COO-$R^6$, -(C=O)- $R^6$, -($C_1$-$C_6$-Alkylen)-$R^7$, -($C_1$-$C_6$-Alkenylen)-$R^7$ , -($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)-Alkyl, ($C_3$-$C_6$)Cycloalkyl, COOH, $COO(C_1-C_6)Alkyl$, $COO(C_3-C_6)Cycloalkyl$, $CONH_2$, $CONH(C_1-C_6)Alkyl$, $CON[(C_1-C_6)Alkyl]2$, $CONH(C_3-C_6)$-Cycloalkyl, $NH_2$, NH-CO-($C_1$-$C_6$)-Alkyl, NH-CO-Phenyl, Pyridyl;

$R^6$ — H, -($C_1$-$C_6$)Alkyl;

$R^7$ — H, (-($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 3 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, S-($C_1$-$C_6$)-Alkyl, SO-($C_1$-$C_6$)-Alkyl, $SO_2$-($C_1$-$C_6$)-Alkyl, ($C_1$-$C_6$)Alkyl, ($C_3$-$C_6$)-Cycloalkyl, COOH, $COO(C_1-C_6)Alkyl$, $COO(C_3-C_6)Cycloalkyl$, $CONH_2$, $CONH(C_1-C_6)Alkyl$, $CON[(C_1-C_6)Alkyl]2$, $CONH(C_3-C_6)Cycloalkyl$, $NH_2$, NH-CO-($C_1$-$C_6$)-Alkyl, NH-CO-Phenyl;

l — 0 oder 1;

m, n — 0;

o — 1;

p — 0 oder 1;

q 0 oder 1;

sowie deren pharmazeutisch verträgliche Salze.

**3.** Verbindungen der Formel I, gemäß Anspruch 1 oder 2, **dadurch gekennzeichnet, daß** darin bedeuten

Z -NH-($C_1$-$C_{12}$-Alkyl)-(C=O)-,
-(C=O)-($C_1$-$C_{12}$-Alkyl)-(C=O)- ,
-(C=O)-Phenyl-(C=O)- ;

$A^1$, $A^2$, $A^3$,$A^4$, unabhängig voneinander einen Aminosäurerest, einen Aminosäurerest der ein- oder mehrfach substituiert ist durch Aminosäure-Schutzgruppen;

E -$SO_2$-$R^4$, -CO-$R^4$;

$R^1$ Phenyl, Thiazolyl, Oxazolyl, wobei die Ringe bis zu 3 mal substituiert sein können mit -($C_1$-$C_6$)-Alkyl;

$R^2$ H, OH, $CH_2OH$, OMe;

$R^3$ H, F, Methyl, OMe;

$R^4$ -($C_5$-$C_{16}$-Alkyl), -($C_0$-$C_{16}$-Alkylen)-$R^5$, -(C=O)-($C_0$-$C_{16}$-Alkylen)-$R^5$, -(C=O)-($C_0$-$C_{16}$-Alkylen)-NH-$R^5$, -($C_1$-$C_8$-Alkenylen)-$R^5$, -($C_1$-$C_8$-Alkinyl), -($C_1$-$C_4$-Alkylen)-$S(O)_{0-2}$-$R^5$, -($C_1$-$C_4$-Alkylen)-O-$R^5$, -($C_1$-$C_4$-Alkylen)-NH-$R^5$;

$R^5$ -COO-$R^6$, -(C=O)-$R^6$ , -($C_1$-$C_7$)-Cycloalkyl, Phenyl, Naphthyl, Thienyl, Thiophenyl, Furanyl, Pyridyl, Pirimidyl, Dihydropyrimidin-2,4-dion-6-yl, Chromanyl, Phthalimidoyl, Thiazolyl, wobei die Ringe bis zu 2 mal substituiert sein können mit F, Cl, Br, OH, $CF_3$, $NO_2$, CN, $OCF_3$, -($C_1$-$C_6$)-Alkyl, O-($C_1$-$C_6$)-Alkyl, COOH, COO($C_1$-$C_6$)Alkyl, $CONH_2$, CONH($C_1$-$C_6$)Alkyl, CON[($C_1$-$C_6$)Alkyl]2, CONH($C_3$-$C_6$)Cycloalkyl, $NH_2$, NH-CO-($C_1$-$C_6$)-Alkyl, NH-CO-Phenyl, Pyridyl;

$R^6$ H, -($C_1$-$C_6$)Alkyl;

l, m, n 0;

o 1;

p 0 oder 1;

q 0 oder 1;

sowie deren pharmazeutisch verträgliche Salze.

**4.** Arzneimittel enthaltend eine oder mehrere der Verbindungen gemäß einem oder mehreren der Ansprüche 1 bis 3.

**5.** Arzneimittel enthaltend eine oder mehrere der Verbindungen gemäß einem oder mehreren der Ansprüche 1 bis 3 und ein oder mehrere lipidsenkende Wirkstoffe.

**6.** Verbindungen gemäß einem oder mehreren der Ansprüche 1 bis 3 zur Anwendung als Medikament zur Prophylaxe oder Behandlung von Lipidstoffwechselstörungen.

**7.** Verbindungen gemäß einem oder mehreren der Ansprüche 1 bis 3 zur Anwendung als Medikament zur Behandlung von Hyperlipidämie.

**8.** Verbindungen gemäß einem oder mehreren der Ansprüche 1 bis 3 zur Anwendung als Medikament zur Prophylaxe oder Behandlung von arteriosklerotischer Erscheinungen.

**9.** Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von Lipidstoffwechselstörungen.

**10.** Verbindungen gemäß einem oder mehreren der Ansprüche 1 bis 3 in Kombination mit mindestens einem weiteren lipidsenkenden Wirkstoff als Medikament zur Behandlung von Hyperlipidämie.

**11.** Verbindungen gemäß einem oder mehreren der Ansprüche 1 bis 3 in Kombination mit mindestens einem weiteren lipidsenkenden Wirkstoff zur Anwendung als Medikament zur Prophylaxe oder Behandlung von arteriosklerotischer Erscheinungen.

**12.** Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

**13.** Verwendung der Verbindungen gemäß einem oder mehreren der Ansprüche 1 bis 3 zur Herstellung eines Medikaments zur Prophylaxe oder Behandlung von Lipidstoffwechselstörungen.

**14.** Verwendung der Verbindungen gemäß einem oder mehreren der Ansprüche 1 bis 3 zur Herstellung eines Medikaments zur Behandlung von Hyperlipidämie.

## Claims

**1.** A compound of the formula I,

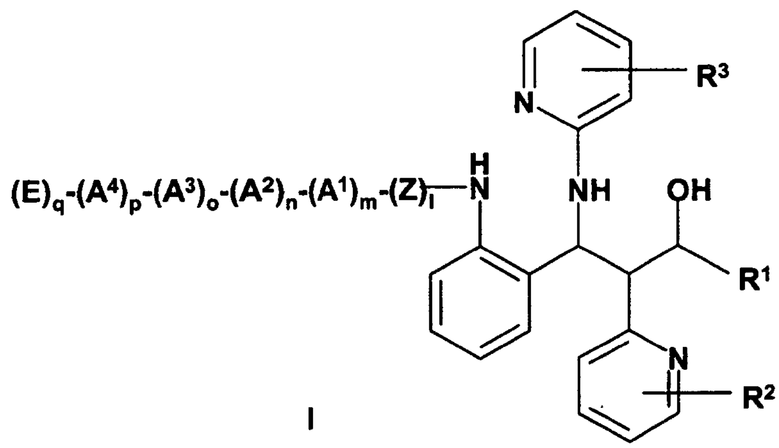

I

Z is $-NH-(C_1-C_{16}\text{-alkyl})-(C=O)-$;
$-(C=O)-(C_1-C_{16}\text{-alkyl})-(C=O)-$ ;
$-(C=O)\text{-phenyl}-(C=O)-$ ;

$A^1$, $A^2$, $A^3$, $A^4$, independently of one another are an amino acid radical, an amino acid radical which is mono- or polysubstituted by amino acid-protective groups;

E is $-SO_2-R^4$, $-CO-R^4$;

$R^1$ is phenyl, thiazolyl, oxazolyl, thienyl, thiophenyl, furanyl, pyridyl, pyrimidyl, it being possible for the rings to be substituted up to 3 times by F, Cl, Br, OH, $CF_3$, $NO_2$, CN, $OCF_3$, $-(C_1-C_6)$alkyl, O-$(C_1-C_6)$-alkyl, S-$(C_1-C_6)$-alkyl, SO-$(C_1-C_6)$-alkyl, $SO_2$-$(C_1-C_6)$-alkyl, $(C_1-C_6)$-alkyl, $(C_3-C_6)$-cycloalkyl, COOH, COO$(C_1-C_6)$alkyl, COO$(C_3-C_6)$cycloalkyl, $CONH_2$, CONH$(C_1-C_6)$alkyl, CON$[(C_1-C_6)\text{alkyl}]_2$, CONH$(C_3-C_6)$cycloalkyl, $NH_2$, NH-CO-$(C_1-C_6)$-alkyl, NH-CO-phenyl;

$R^2$ is H, OH, $CH_2OH$, OMe;

$R^3$ is H, F, methyl, OMe;

$R^4$ is $-(C_5-C_{16}$-alkyl), $-(C_0-C_{16}$-alkylene)-$R^5$, -(C=O)-$(C_0-C_{16}$-alkylene)-$R^5$, -(C=O)-$(C_0-C_{16}$-alkylene) -NH-$R^5$, $-(C_1-C_8$-alkenylene)-$R^5$, $-(C_1-C_8$-alkynyl), $-(C_1-C_4$-alkylene)-$S(O)_{0-2}$ -$R^5$, $-(C_1-C_4$-alkylene)-O -$R^5$, $-(C_1-C_4$-alkylene)-NH -$R^5$;

$R^5$ is -COO-$R^6$, -(C=O)- $R^6$ , $-(C_1-C_6$-alkylene)-$R^7$, $-(C_1-C_6$-alkenylene)-$R^7$ , $-(C_1-C_7)$-cycloalkyl, phenyl, naphthyl, thienyl, thiophenyl, furanyl, pyridyl, pyrimidyl, dihydropyrimidine-2,4-dion-6-yl, chromanyl, phthalimidoyl, thiazolyl, it being possible for the rings to be substituted up to 3 times by F, Cl, Br, OH, $CF_3$, $NO_2$, CN, $OCF_3$, $-(C_1-C_6)$-alkyl, O-$(C_1-C_6)$-alkyl, S-$(C_1-C_6)$-alkyl, SO-$(C_1-C_6)$-alkyl, $SO_2$-$(C_1-C_6)$-alkyl, $(C_1-C_6)$-alkyl, $(C_3-C_6)$-cycloalkyl, COOH, COO$(C_1-C_6)$ alkyl, COO$(C_3-C_6)$cycloalkyl, $CONH_2$, CONH$(C_1-C_6)$alkyl, CON$[(C_1-C_6)alkyl]_2$, CONH$(C_3-C_6)$ cycloalkyl, $NH_2$, NH-CO-$(C_1-C_6)$alkyl, NH-CO-phenyl, pyridyl;

$R^6$ is H, $-(C_1-C_6)$alkyl;

$R^7$ is H, $-(C_1-C_7)$-cycloalkyl, phenyl, naphthyl, thienyl, thiophenyl, furanyl, pyridyl, pyrimidyl, dihydropyrimidine-2,4-dion-6-yl, chromanyl, phthalimidoyl, thiazolyl, it being possible for the rings to be substituted up to 3 times by F, Cl, Br, OH, $CF_3$, $NO_2$, CN, $OCF_3$, $-(C_1-C_6)$-alkyl, O-$(C_1-C_6)$-alkyl, S-$(C_1-C_6)$alkyl, SO-$(C_1-C_6)$-alkyl, $SO_2$-$(C_1-C_6)$-alkyl, $(C_1-C_6)$-alkyl, $(C_3-C_6)$-cycloalkyl, COOH, COO$(C_1-C_6)$alkyl, COO$(C_3-C_6)$cycloalkyl, $CONH_2$, CONH$(C_1-C_6)$alkyl, CON$[(C_1-C_6)alkyl]_2$, CONH$(C_3-C_6)$cycloalkyl, $NH_2$, NH-CO-$(C_1-C_6)$-alkyl, NH-CO-phenyl;

l, q, m, n, o, p independently of one another are 0 or 1, where l+q+m+n+o+p is greater than or equal to 1;

and pharmaceutically tolerated salts thereof.

**2.** A compound of the formula I as claimed in claim 1, wherein

Z is -NH-$(C_1-C_{16}$-alkyl)-(C=O)-,
-(C=O)-$(C_1-C_{16}$-alkyl)-(C=O)- ,
-(C=O)-phenyl-(C=O)- ;

$A^1$, $A^2$, $A^3$, $A^4$ independently of one another are an amino acid radical, an amino acid radical which is mono- or polysubstituted by amino acid-protective groups;

E is -$SO_2$-$R^4$, -CO-$R^4$;

$R^1$ is phenyl, thiazolyl, oxazolyl, thienyl, thiophenyl, furanyl, pyridyl, pyrimidyl, it being possible for the rings to be substituted up to 3 times by F, Cl, Br, OH, $CF_3$, $NO_2$, CN, $OCF_3$, $-(C_1-C_6)$alkyl, O-$(C_1-C_6)$-alkyl, S-$(C_1-C_6)$-alkyl, SO-$(C_1-C_6)$-alkyl, $SO_2$-$(C_1-C_6)$-alkyl, $(C_1-C_6)$-alkyl, $(C_3-C_6)$-cycloalkyl, COOH, COO$(C_1-C_6)$alkyl, COO$(C_3-C_6)$cycloalkyl, $CONH_2$, CONH$(C_1-C_6)$alkyl, CON$[(C_1-C_6)alkyl]_2$, CONH$(C_3-C_6)$cycloalkyl, $NH_2$, NH-CO-$(C_1-C_6)$-alkyl, NH-CO-phenyl,;

$R^2$ is H, OH, $CH_2OH$, OMe;

$R^3$ is H, F, methyl, OMe;

$R^4$ is $-(C_5-C_{16}$-alkyl), $-(C_0-C_{16}$-alkylene)-$R^5$, -(C=O)-$(C_0-C_{16}$-alkylene)-$R^5$, -(C=O)-$(C_0-C_{16}$-alkylene) -NH-$R^5$, $-(C_1-C_8$-alkenylene)-$R^5$, $-(C_1-C_8$-alkynyl), $-(C_1-C_4$-alkylene)-$S(O)_{0-2}$ -$R^5$, $-(C_1-C_4$-alkylene)-O-$R^5$, $-(C_1-C_4$-alkylene)-NH -$R^5$;

$R^5$ is -COO-$R^6$, -(C=O)- $R^6$ , $-(C_1-C_6$-alkylene)-$R^7$, $-(C_1-C_6$-alkenylene)-$R^7$ , $-(C_1-C_7)$-cycloalkyl, phenyl, naphthyl, thienyl, thiophenyl, furanyl, pyridyl, pyrimidyl, dihydropyrimidine-2,4-dion-6-yl, chromanyl, phthalimidoyl, thiazolyl, it being possible for the rings to be substituted up to 3 times by F, Cl, Br, OH, $CF_3$, $NO_2$, CN, $OCF_3$, $-(C_1-C_6)$-alkyl, O-$(C_1-C_6)$alkyl, S-$(C_1-C_6)$-alkyl,

SO-($C_1$-$C_6$)-alkyl, $SO_2$-($C_1$-$C_6$)-alkyl, ($C_1$-$C_6$)-alkyl, ($C_3$-$C_6$)-cycloalkyl, COOH, COO($C_1$-$C_6$) alkyl, COO($C_3$-$C_6$)cycloalkyl, $CONH_2$, CONH($C_1$-$C_6$)alkyl, CON[($C_1$-$C_6$)alkyl]$_2$, CONH($C_3$-$C_6$)cycloalkyl, $NH_2$, NH-CO-($C_1$-$C_6$)-alkyl, NH-CO-phenyl, pyridyl;

$R^6$ is H, -($C_1$-$C_6$)alkyl;

$R^7$ is H, -($C_1$-$C_7$)-cycloalkyl, phenyl, naphthyl, thienyl, thiophenyl, furanyl, pyridyl, pyrimidyl, dihydropyrimidine-2,4-dion-6-yl, chromanyl, phthalimidoyl, thiazolyl, it being possible for the rings to be substituted up to 3 times by F, Cl, Br, OH, $CF_3$, $NO_2$, CN, $OCF_3$, -($C_1$-$C_6$)-alkyl, O-($C_1$-$C_6$)-alkyl, S-($C_1$-$C_6$)alkyl, SO-($C_1$-$C_6$)-alkyl, $SO_2$-($C_1$-$C_6$)-alkyl, ($C_1$-$C_6$)-alkyl, ($C_3$-$C_6$)-cycloalkyl, COOH, COO($C_1$-$C_6$)alkyl, COO($C_3$-$C_6$)cycloalkyl, $CONH_2$, CONH($C_1$-$C_6$)alkyl, CON[($C_1$-$C_6$)alkyl]$_2$, CONH($C_3$-$C_6$)cycloalkyl, $NH_2$, NH-CO-($C_1$-$C_6$)alkyl, NH-CO-phenyl;

I is 0 or 1;

m, n are 0;

o is 1;

p is 0 or 1;

q is 0 or 1;

and pharmaceutically tolerated salts thereof.

**3.** A compound of the formula I as claimed in claim 1 or 2, wherein

Z is -NH-($C_1$-$C_{12}$-alkyl)-(C=O)-,
-(C=O)-($C_1$-$C_{12}$-alkyl)-(C=O)- ,
-(C=O)-phenyl-(C=O)- ;

$A^1$, $A^2$, $A^3$, $A^4$ independently of one another are an amino acid radical, an amino acid radical which is mono- or polysubstituted by amino acid-protective groups;

E is -$SO_2$-$R^4$, -CO-$R^4$;

$R^1$ is phenyl, thiazolyl, oxazolyl, it being possible for the rings to be substituted up to 3 times by -($C_1$-$C_6$)-alkyl;

$R^2$ is H, OH, $CH_2OH$, OMe;

$R^3$ is H, F, methyl, OMe;

$R^4$ is -($C_5$-$C_{16}$-alkyl), -($C_0$-$C_{16}$-alkylene)-$R^5$, -(C=O)-($C_0$-$C_{16}$-alkylene)-$R^5$, -(C=O)-($C_0$-$C_{16}$-alkylene) -NH-$R^5$, -($C_1$-$C_8$-alkenylene)-$R^5$, -($C_1$-$C_8$-alkynyl), -($C_1$-$C_4$-alkylene)-S(O)$_{0-2}$ -$R^5$, -($C_1$-$C_4$-alkylene)-O -$R^5$, -($C_1$-$C_4$-alkylene)-NH -$R^5$;

$R^5$ is -COO-$R^6$, -(C=O)- $R^6$ , -($C_1$-$C_7$)-cycloalkyl, phenyl, naphthyl, thienyl, thiophenyl, furanyl, pyridyl, pyrimidyl, dihydropyrimidine-2,4-dion-6-yl, chromanyl, phthalimidoyl, thiazolyl, it being possible for the rings to be substituted up to twice by F, Cl, Br, OH, $CF_3$, $NO_2$, CN, $OCF_3$, -($C_1$-$C_6$)-alkyl, O-($C_1$-$C_6$)-alkyl, COOH, COO($C_1$-$C_6$)alkyl, $CONH_2$, CONH($C_1$-$C_6$)alkyl, CON[($C_1$-$C_6$)alkyl]$_2$, CONH($C_3$-$C_6$)cycloalkyl, $NH_2$, NH-CO-($C_1$-$C_6$)-alkyl, NH-CO-phenyl, pyridyl;

$R^6$ is H, -($C_1$-$C_6$)alkyl;

I, m, n are 0;

o is 1;

p is 0 or 1;

q is 0 or 1;

and pharmaceutically tolerated salts thereof.

**4.** A pharmaceutical comprising one or more compounds as claimed in one or more of claims 1 to 3.

**5.** A pharmaceutical comprising one or more compounds as claimed in one or more of claims 1 to 3 and one or more lipid-lowering active compounds.

**6.** A compound as claimed in one or more of claims 1 to 3 for use as a pharmaceutical for the prophylaxis or treatment of disturbances in lipid metabolism.

**7.** A compound as claimed in one or more of claims 1 to 3 for use as a pharmaceutical for the treatment of hyperlipidemia.

**8.** A compound as claimed in one or more of claims 1 to 3 for use as a pharmaceutical for the prophylaxis or treatment of arteriosclerotic symptoms.

**9.** A compound as claimed in one or more of claims 1 to 3 in combination with at least one further lipid-lowering active compound for use as a pharmaceutical for the prophylaxis or treatment of disturbances in lipid metabolism.

**10.** A compound as claimed in one or more of claims 1 to 3 in combination with at least one further lipid-lowering active compound as a pharmaceutical for treatment of hyperlipidemia.

**11.** A compound as claimed in one or more of claims 1 to 3 in combination with at least one further lipid-lowering active compound for use as a pharmaceutical for the prophylaxis or treatment of arteriosclerotic symptoms.

**12.** A process for the preparation of a pharmaceutical comprising one or more compounds according to one or more of claims 1 to 3, which comprises mixing the active compound with a pharmaceutically suitable excipient and bringing this mixture into a form suitable for administration.

**13.** The use of a compound as claimed in one or more of claims 1 to 3 for the preparation of a pharmaceutical for the prophylaxis or treatment of disturbances in lipid metabolism.

**14.** The use of a compound as claimed in one or more of claims 1 to 3 for the preparation of a pharmaceutical for the treatment of hyperlipidemia.

## Revendications

**1.** Composés de formule I,

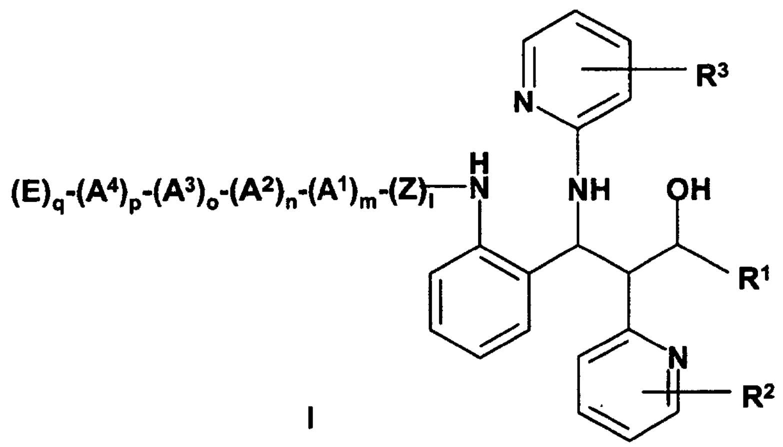

I

Z représente un groupe
-NH- (alkyl en $C_1$-$C_{16}$)-(C=O)- ;
-(C=O)-(alkyl en $C_1$-$C_{16}$) - (C=O) - ;
-(C=O)-phényl-(C=O) ;

$A^1$, $A^2$, $A^3$, $A^4$, indépendamment les uns des autres, représentent un reste acide aminé, un reste acide aminé qui est substitué une ou plusieurs fois par des groupes protecteurs d'acide aminé ;

E représente un groupe -$SO_2$-$R^4$, -CO-$R^4$ ;

$R^1$ représente un groupe phényle, thiazolyle, oxazolyle, thiényle, thiophényle, furanyle, pyridyle, pirimidyle, les cycles pouvant être substitués jusqu'à 3 fois par des substituants F, Cl, Br, OH, $CF_3$, $NO_2$, CN, $OCF_3$, alkyle en $C_1$-$C_6$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$ CON(alkyle en $C_1$-$C_6$)$_2$, CONH-cycloalkyle en $C_3$-$C_6$, $NH_2$, NH-CO-alkyle en $C_1$-$C_6$, NH-CO-phényle ;

$R^2$ H, OH, $CH_2OH$, OMe ;

$R^3$ H, F, méthyle, OMe ;

$R^4$ représente un groupe alkyle en $C_5$-$C_{16}$, -(alkylène en $C_0$-$C_{16}$)-$R^5$, -(C=O)-(alkylène en $C_0$-$C_{16}$) -$R^5$, -(C=O)-(alkylène en $C_0$-$C_{16}$)-NH-$R^5$, -(alcénylène en $C_1$-$C_8$)-$R^5$, alcynyle en $C_1$-$C_8$, -(alkylène en $C_1$-$C_4$)-S(O)$_{0-2}$-$R^5$, -(alkylène en $C_1$-$C_4$)-O-$R^5$-, -(alkylène en $C_1$-$C_4$)-NH-$R^5$ ;

$R^5$ représente un groupe -COO-$R^6$, -(C=O) -$R^6$, (alkylène en $C_1$-$C_6$) -$R^7$, -(alcénylène en $C_1$-$C_6$) -$R^7$, cycloalkyle en $C_1$-$C_7$, phényle, naphtyle, thiényle, thiophényle, furanyle, pyridyle, pirimidyle, dihydropyrimidine-2,4-dion-6-yle, chromanyle, phtalimidoyle, thiazolyle, les cycles pouvant être substitués jusqu'à 3 fois par des substituants F, Cl, Br, OH, $CF_3$, $NO_2$, CN, $OCF_3$, alkyle en $C_1$-$C_6$, -O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$, CON (alkyle en $C_1$-$C_6$)$_2$, CONH-cycloalkyle en $C_3$-$C_6$, $NH_2$, NH-CO-alkyle en $C_1$-$C_6$, NH-CO-phényle, pyridyle ;

$R^6$ représente un atome d'hydrogène, un groupe alkyle en $C_1$-$C_6$ ;

$R^7$ représente un atome d'hydrogène, un groupe cycloalkyle en $C_1$-$C_7$, phényle, naphtyle, thiényle, thiophényle, furanyle, pyridyle, pirimidyle, dihydropyrimidine-2,4-dion-6-yle, chromanyle, phtalimidoyle, thiazolyle, les cycles pouvant être substitués jusqu'à 3 fois par des substituants F, Cl, Br, OH, $CF_3$, $NO_2$, CN, $OCF_3$, alkyle en $C_1$-$C_6$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$, CON (alkyle en $C_1$-$C_6$)$_2$, CONH-cycloalkyle en $C_3$-$C_6$, $NH_2$, NH-CO-alkyle en $C_1$-$C_6$, NH-CO-phényle ;

l, q, m, n, o, p valent indépendamment les uns des autres 0 ou 1 avec l+q+m+n+o+p supérieure ou égale à 1 ;

ainsi que leurs sels tolérés en pharmacie.

2. Composés de formule I, selon la revendication 1, **caractérisés en ce que**

Z représente un groupe

-NH-(al kyl en $C_1$-$C_{16}$)-(C=O)- ;
-(C=O)-(alkyl en $C_1$-$C_{16}$)-(C=O)- ;
-(C=O)-phényl-(C=O) ;

| | |
|---|---|
| $A^1$, $A^2$, $A^3$, $A^4$, | indépendamment les uns des autres, représentent un reste acide aminé, un reste acide aminé qui est substitué une ou plusieurs fois par des groupes protecteurs d'acide aminé ; |
| E | représente un groupe -$SO_2$-$R^4$, -CO-$R^4$ ; |
| $R^1$ | représente un groupe phényle, thiazolyle, oxazolyle, thiényle, thiophényle, furanyle, pyridyle, pirimidyle, les cycles pouvant être substitués jusqu'à 3 fois par des substituants F, Cl, Br, OH, $CF_3$, $NO_2$, CN, OCF3, alkyle en $C_1$-$C_6$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$ CON(alkyle en $C_1$-$C_6$)$_2$, CONH-cycloalkyle en $C_3$-$C_6$, $NH_2$, NH-CO-alkyle en $C_1$-$C_6$, NH-CO-phényle ; |
| $R^2$ | H, OH, $CH_2OH$, OMe ; |
| $R^3$ | H, F, méthyle, OMe ; |
| $R^4$ | représente un groupe alkyle en $C_5$-$C_{16}$, -(alkylène en $C_0$-$C_{16}$)-$R^5$, -(C=O)-(alkylène en $C_0$-$C_{16}$) -$R^5$, -(C=O)-(alkylène en $C_0$-$C_{16}$)-NH-$R^5$, -(alcénylène en $C_1$-$C_8$)-$R^5$, alcynyle en $C_1$-$C_8$, (alkylène en $C_1$-$C_4$)-S(O)$_{0-2}$-$R^5$, -(alkylène en $C_1$-$C_4$)-O-$R^5$-, -(alkylène en $C_1$-$C_4$)-NH-$R^5$ ; |
| $R^5$ | représente un groupe -COO-$R^6$, -(C=O)-$R^6$, -(alkylène en $C_1$-$C_6$)-$R^7$, -(alcénylène en $C_1$-$C_6$)-$R^7$, cycloalkyle en $C_1$-$C_7$, phényle, naphtyle, thiényle, thiophényle, furanyle, pyridyle, pirimidyle, dihydropyrimidine-2,4-dion-6-yle, chromanyle, phtalimidoyle, thiazolyle, les cycles pouvant être substitués jusqu'à 3 fois par des substituants F, Cl, Br, OH, $CF_3$, $NO_2$, CN, $OCF_3$, alkyle en $C_1$-$C_6$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$, CON(alkyle en $C_1$-$C_6$)$_2$, CONH-cycloalkyle en $C_3$-$C_6$, $NH_2$, NH-CO-alkyle en $C_1$-$C_6$, NH-CO-phényle, pyridyle ; |
| $R^6$ | représente un atome d'hydrogène, un groupe alkyle en $C_1$-$C_6$ ; |
| $R^7$ | représente un atome d'hydrogène, un groupe cycloalkyle en $C_1$-$C_7$, phényle, naphtyle, thiényle, thiophényle, furanyle, pyridyle, pirimidyle, dihydropyrimidine-2,4-dion-6-yle, chromanyle, phtalimidoyle, thiazolyle, les cycles pouvant être substitués jusqu'à 3 fois par des substituants F, Cl, Br, OH, $CF_3$, $NO_2$, CN, $OCF_3$, alkyle en $C_1$-$C_6$, O-alkyle en $C_1$-$C_6$, S-alkyle en $C_1$-$C_6$, SO-alkyle en $C_1$-$C_6$, $SO_2$-alkyle en $C_1$-$C_6$, alkyle en $C_1$-$C_6$, cycloalkyle en $C_3$-$C_6$, COOH, COO-alkyle en $C_1$-$C_6$, COO-cycloalkyle en $C_3$-$C_6$, $CONH_2$, CONH-alkyle en $C_1$-$C_6$, CON(alkyle en $C_1$-$C_6$)$_2$, CONH-cycloalkyle en $C_3$-$C_6$, $NH_2$, NH-CO-alkyle en $C_1$-$C_6$, NH-CO-phényle ; |
| l | vaut 0 ou 1 ; |
| m, n | valent 0 ; |
| o | vaut 1 ; |
| p | vaut 0 ou 1 ; |
| q | vaut 0 ou 1 ; |

ainsi que leurs sels tolérés en pharmacie.

3. Composés de formule I, selon l'une des revendications 1 ou 2, **caractérisés en ce que**

| | |
|---|---|
| Z | représente un groupe<br>-NH-(alkyl en $C_1$-$C_{12}$)-(C=O)- ;<br>-(C=O)-(alkyl en $C_1$-$C_{12}$)-(C=O)- ;<br>-(C=O)-phényl-(C=O) ; |
| $A^1$, $A^2$, $A^3$, $A^4$, | indépendamment les uns des autres, représentent un reste acide aminé, un restes acide aminé qui est substitué une ou plusieurs fois par des groupes protecteurs d'acide aminé ; |
| E | représente un groupe -$SO_2$-$R^4$, -CO-$R^4$ ; |
| $R^1$ | représente un groupe phényle, thiazolyle, oxazolyle, les cycles pouvant être substitués jusqu'à 3 fois par des substituants alkyle en $C_1$-$C_6$ ; |
| $R^2$ | H, OH, $CH_2OH$, OMe ; |
| $R^3$ | H, F, méthyle, OMe ; |
| $R^4$ | représente un groupe alkyle en $C_5$-$C_{16}$, -(alkylène en $C_0$-$C_{16}$)-$R^5$, -(C=O)-(alkylène en $C_0$-$C_{16}$) -$R^5$, -(C=O)-(alkylène en $C_0$-$C_{16}$)-NH-$R^5$, -(alcénylène en $C_1$-$C_8$)-$R^5$, alcynyle en $C_1$-$C_8$, (alkylène en $C_1$-$C_4$)-S(O)$_{0-2}$-$R^5$, -(alkylène en $C_1$-$C_4$)-O-$R^5$-, -(alkylène en $C_1$-$C_4$)-NH-$R^5$ ; |
| $R^5$ | représente un groupe -COO-$R^6$, -(C=O)-$R^6$, cycloalkyle en $C_1$-$C_7$, phényle, naphtyle, thiényle, |

| | |
|---|---|
| | thiophényle, furanyle, pyridyle, pirimidyle, dihydropyrimidine-2,4-dion-6-yle, chromanyle, phtalimidoyle, thiazolyle, les cycles pouvant être substitués jusqu'à 2 fois par des substituants F, Cl, Br, OH, $CF_3$, $NO_2$, CN, $OCF_3$, alkyle en $C_1$-$C_6$, O-alkyle en $C_1$-$C_6$, COOH, COO-alkyle en $C_1$-$C_6$, $CONH_2$, CONH-alkyle en $C_1$-$C_6$, CON(alkyle en $C_1$-$C_6$)$_2$, CONH-cycloalkyle en $C_3$-$C_6$, $NH_2$, NH-CO- (alkyle en $C_1$-$C_6$), NH-CO-phényle, pyridyle ; |
| $R^6$ | représente un atome d'hydrogène, un groupe alkyle en $C_1$-$C_6$ ; |
| l, m, n | valent 0 ; |
| o | vaut 1 ; |
| p | vaut 0 ou 1 ; |
| q | vaut 0 ou 1 ; |

ainsi que leurs sels tolérés en pharmacie.

**4.** Médicament renfermant un ou plusieurs composés selon une ou plusieurs des revendications 1 à 3.

**5.** Médicament renfermant un ou plusieurs composés selon une ou plusieurs des revendications 1 à 3 et un ou plusieurs principes actifs hypolipidémiants.

**6.** Composés selon une ou plusieurs des revendications 1 à 3 pour l'utilisation comme médicament pour la prévention ou le traitement des troubles de métabolisme des lipides.

**7.** Composés selon une' ou plusieurs des revendications 1 à 3 pour l'utilisation comme médicament pour le traitement d'hyperlipidémie.

**8.** Composés selon une ou plusieurs des revendications 1 à 3 pour l'utilisation comme médicament pour la prévention ou le traitement de phénomènes artériosclérotiques.

**9.** Composés selon une ou plusieurs des revendications 1 à 3 en combinaison avec au moins un autre principe actif hypolipidémiant pour l'utilisation comme médicament pour la prévention ou le traitement de troubles du métabolisme des lipides.

**10.** Composés selon une où plusieurs des revendications 1 à 3 en combinaison avec au moins un autre principe actif hypolipidémiant pour l'utilisation comme médicament pour la prévention ou le traitement d'hyperlipidémie.

**11.** Composés selon une ou plusieurs des revendications 1 à 3 en combinaison avec au moins un autre principe actif hypolipidémiant pour l'utilisation comme médicament pour la prévention ou le traitement de phénomènes artériosclérotiques.

**12.** Procédé pour la préparation d'un médicament renfermant un ou plusieurs des composés selon une ou plusieurs des revendications 1 à 3, **caractérisé en ce qu'**on mélange le principe actif avec un véhicule pharmaceutique approprié et on met ce mélange en forme appropriée pour l'administration.

**13.** Utilisation des composés selon une ou plusieurs des revendications 1 à 3 pour la préparation d'un médicament pour la prévention ou le traitement de troubles du métabolisme des lipides.

**14.** Utilisation des composés selon une ou plusieurs des revendications 1 à 3 pour la préparation d'un médicament pour le traitement d'hyperlipidémie.